# EUROPEAN PATENT SPECIFICATION

(11) **EP 1 896 599 B1**
(45) Date of publication and mention of the grant of the patent: **05.08.2009**
(21) Application number: 06767909.2
(22) Date of filing: 29.06.2006
(51) Int. Cl.: C12P 13/08, A23K 1/00

(54) **METHOD FOR PRODUCING L-THREONINE**
VERFAHREN ZUR HERSTELLUNG VON L-THREONIN
PROCEDE DE PRODUCTION DE L-THREONINE

(30) Priority: 29.06.2005 JP 2005189106; 24.04.2006 JP 2006119334
(43) Date of publication of application: 12.03.2008
(73) Proprietor: Ajinomoto Co., Inc., Tokyo 104-8315 (JP)
(72) Inventor: JOE, Yuji, AJINOMOTO CO., INC., Kawasaki-shi, Kanagawa, 210-8681 (JP); KOYAMA, Naoto, AJINOMOTO CO., INC., Kawasaki-shi, Kanagawa, 210-8681 (JP); KATO, Naoto, AJINOMOTO CO., INC., Kawasaki-shi, Kanagawa, 210-8681 (JP); TSUJI, Yuichiro, AJINOMOTO CO., INC., Kawasaki-shi, Kanagawa, 210-8681 (JP)
(74) Representative: HOFFMANN EITLE
(86) International application number: PCT/JP2006/313450
(87) International publication number: WO 2007/001097

(56) References cited:
- WO-A-20/05049808
- US-A- 4 400 467
- US-A- 5 763 230
- US-A1- 2005 025 878
- US-B1- 6 261 825

### SEQUENCE LISTING

<110> Ajinomoto Co., Inc.
<120> Method for producing L-threonine
<130> C586-C6078
<150> JP2005-189106
   <151> 2005-06-29
<150> JP2006-119334
   <151> 2006-04-24
<160> 6
<170> PatentIn version 3.1
<210> 1
   <211> 5040
   <212> DNA
   <213> Escherichia coli
<220>
   <221> promoter
   <222> (71)..(99)
   <223> factor Sigma 70; predicted +1 start at 106
<220>
   <221> promoter
   <222> (104)..(132)
   <223> factor Sigma 70; predicted +1 start at 139
<220>
   <221> promoter
   <222> (139)..(219)
   <223> factor Sigma 70; predicted +1 start at 219
<220>
   <221> CDS
   <222> (190)..(255)
   <223> leader peptide
<220>
   <221> attenuator
   <222> (273)..(307)
<220>
   <221> CDS
   <222> (337)..(2799)
   <223> thrA
<220>
   <221> CDS
   <222> (2801)..(3733)
   <223> thrB
<220>
   <221> CDS
   <222> (3734)..(5020)
   <223> thrC
<400> 1
   agcttttcat tctgactgca acgggcaata tgtctctgtg tggattaaaa aaagagtgtc      60
   tgatagcagc ttctgaactg gttacctgcc gtgagtaaat taaaatttta ttgacttagg     120
   tcactaaata ctttaaccaa tataggcata gcgcacagac agataaaaat tacagagtac     180
   acaacatcc atg aaa cgc att agc acc acc att acc acc acc atc acc att     231
             Met Lys Arg Ile Ser Thr Thr Ile Thr Thr Thr Ile Thr Ile
             1               5                   10
   acc aca ggt aac ggt gcg ggc tga cgcgtacagg aaacacagaa aaaagcccgc    285
   Thr Thr Gly Asn Gly Ala Gly
   15                  20
   acctgacagt gcgggctttt tttttcgacc aaaggtaacg aggtaacaac c atg cga     342
                                                            Met Arg
   gtg ttg aag ttc ggc ggt aca tca gtg gca aat gca gaa cgt ttt ctg      390
   Val Leu Lys Phe Gly Gly Thr Ser Val Ala Asn Ala Glu Arg Phe Leu
       25                  30                  35
   cgt gtt gcc gat att ctg gaa agc aat gcc agg cag ggg cag gtg gcc      438
   Arg Val Ala Asp Ile Leu Glu Ser Asn Ala Arg Gln Gly Gln Val Ala
   40                  45                  50                  55
   acc gtc ctc tct gcc ccc gcc aaa atc acc aac cac ctg gtg gcg atg      486
   Thr Val Leu Ser Ala Pro Ala Lys Ile Thr Asn His Leu Val Ala Met
                   60                  65                  70
   att gaa aaa acc att agc ggc cag gat gct tta ccc aat atc agc gat      534
   Ile Glu Lys Thr Ile Ser Gly Gln Asp Ala Leu Pro Asn Ile Ser Asp
               75                  80                  85
   gcc gaa cgt att ttt gcc gaa ctt ttg acg gga ctc gcc gcc gcc cag      582
   Ala Glu Arg Ile Phe Ala Glu Leu Leu Thr Gly Leu Ala Ala Ala Gln
           90                  95                  100
   ccg ggg ttc ccg ctg gcg caa ttg aaa act ttc gtc gat cag gaa ttt      630
   Pro Gly Phe Pro Leu Ala Gln Leu Lys Thr Phe Val Asp Gln Glu Phe
       105                 110                 115
   gcc caa ata aaa cat gtc ctg cat ggc att agt ttg ttg ggg cag tgc      678
   Ala Gln Ile Lys His Val Leu His Gly Ile Ser Leu Leu Gly Gln Cys
   120                 125                 130                 135
   ccg gat agc atc aac gct gcg ctg att tgc cgt ggc gag aaa atg tcg      726
   Pro Asp Ser Ile Asn Ala Ala Leu Ile Cys Arg Gly Glu Lys Met Ser
                   140                 145                 150
   atc gcc att atg gcc ggc gta tta gaa gcg cgc ggt cac aac gtt act      774
   Ile Ala Ile Met Ala Gly Val Leu Glu Ala Arg Gly His Asn Val Thr
               155                 160                 165
   gtt atc gat ccg gtc gaa aaa ctg ctg gca gtg ggg cat tac ctc gaa      822
   Val Ile Asp Pro Val Glu Lys Leu Leu Ala Val Gly His Tyr Leu Glu
           170                 175                 180
   tct acc gtc gat att gct gag tcc acc cgc cgt att gcg gca agc cgc      870
   Ser Thr Val Asp Ile Ala Glu Ser Thr Arg Arg Ile Ala Ala Ser Arg
       185                 190                 195
   att ccg gct gat cac atg gtg ctg atg gca ggt ttc acc gcc ggt aat      918
   Ile Pro Ala Asp His Met Val Leu Met Ala Gly Phe Thr Ala Gly Asn
   200                 205                 210                 215
   gaa aaa ggc gaa ctg gtg gtg ctt gga cgc aac ggt tcc gac tac tct      966
   Glu Lys Gly Glu Leu Val Val Leu Gly Arg Asn Gly Ser Asp Tyr Ser
                   220                 225                 230
   gct gcg gtg ctg gct gcc tgt tta cgc gcc gat tgt tgc gag att tgg      1014
   Ala Ala Val Leu Ala Ala Cys Leu Arg Ala Asp Cys Cys Glu Ile Trp
               235                 240                 245
   acg gac gtt gac ggg gtc tat acc tgc gac ccg cgt cag gtg ccc gat      1062
   Thr Asp Val Asp Gly Val Tyr Thr Cys Asp Pro Arg Gln Val Pro Asp
           250                 255                 260
   gcg agg ttg ttg aag tcg atg tcc tac cag gaa gcg atg gag ctt tcc      1110
   Ala Arg Leu Leu Lys Ser Met Ser Tyr Gln Glu Ala Met Glu Leu Ser
       265                 270                 275
   tac ttc ggc gct aaa gtt ctt cac ccc cgc acc att acc ccc atc gcc      1158
   Tyr Phe Gly Ala Lys Val Leu His Pro Arg Thr Ile Thr Pro Ile Ala
   280                 285                 290                 295
   cag ttc cag atc cct tgc ctg att aaa aat acc gga aat cct caa gca      1206
   Gln Phe Gln Ile Pro Cys Leu Ile Lys Asn Thr Gly Asn Pro Gln Ala
                   300                 305                 310
   cca ggt acg ctc att ggt gcc agc cgt gat gaa gac gaa tta ccg gtc      1254
   Pro Gly Thr Leu Ile Gly Ala Ser Arg Asp Glu Asp Glu Leu Pro Val
               315                 320                 325
   aag ggc att tcc aat ctg aat aac atg gca atg ttc agc gtt tct ggt      1302
   Lys Gly Ile Ser Asn Leu Asn Asn Met Ala Met Phe Ser Val Ser Gly
           330                 335                 340
   ccg ggg atg aaa ggg atg gtc ggc atg gcg gcg cgc gtc ttt gca gcg      1350
   Pro Gly Met Lys Gly Met Val Gly Met Ala Ala Arg Val Phe Ala Ala
       345                 350                 355
   atg tca cgc gcc cgt att tcc gtg gtg ctg att acg caa tca tct tcc      1398
   Met Ser Arg Ala Arg Ile Ser Val Val Leu Ile Thr Gln Ser Ser Ser
   360                 365                 370                 375
   gaa tac agc atc agt ttc tgc gtt cca caa agc gac tgt gtg cga gct      1446
   Glu Tyr Ser Ile Ser Phe Cys Val Pro Gln Ser Asp Cys Val Arg Ala
                   380                 385                 390
   gaa cgg gca atg cag gaa gag ttc tac ctg gaa ctg aaa gaa ggc tta      1494
   Glu Arg Ala Met Gln Glu Glu Phe Tyr Leu Glu Leu Lys Glu Gly Leu
               395                 400                 405
   ctg gag ccg ctg gca gtg acg gaa cgg ctg gcc att atc tcg gtg gta      1542
   Leu Glu Pro Leu Ala Val Thr Glu Arg Leu Ala Ile Ile Ser Val Val
           410                 415                 420
   ggt gat ggt atg cgc acc ttg cgt ggg atc tcg gcg aaa ttc ttt gcc      1590
   Gly Asp Gly Met Arg Thr Leu Arg Gly Ile Ser Ala Lys Phe Phe Ala
       425                 430                 435
   gca ctg gcc cgc gcc aat atc aac att gtc gcc att gct cag gga tct      1638
   Ala Leu Ala Arg Ala Asn Ile Asn Ile Val Ala Ile Ala Gln Gly Ser
   440                 445                 450                 455
   tct gaa cgc tca atc tct gtc gtg gta aat aac gat gat gcg acc act      1686
   Ser Glu Arg Ser Ile Ser Val Val Val Asn Asn Asp Asp Ala Thr Thr
                   460                 465                 470
   ggc gtg cgc gtt act cat cag atg ctg ttc aat acc gat cag gtt atc      1734
   Gly Val Arg Val Thr His Gln Met Leu Phe Asn Thr Asp Gln Val Ile
               475                 480                 485
   gaa gtg ttt gtg att ggc gtc ggt ggc gtt ggc ggt gcg ctg ctg gag      1782
   Glu Val Phe Val Ile Gly Val Gly Gly Val Gly Gly Ala Leu Leu Glu
           490                 495                 500
   caa ctg aag cgt cag caa agc tgg ctg aag aat aaa cat atc gac tta      1830
   Gln Leu Lys Arg Gln Gln Ser Trp Leu Lys Asn Lys His Ile Asp Leu
       505                 510                 515
   cgt gtc tgc ggt gtt gcc aac tcg aag gct ctg ctc acc aat gta cat      1878
   Arg Val Cys Gly Val Ala Asn Ser Lys Ala Leu Leu Thr Asn Val His
   520                 525                 530                 535
   ggc ctt aat ctg gaa aac tgg cag gaa gaa ctg gcg caa gcc aaa gag      1926
   Gly Leu Asn Leu Glu Asn Trp Gln Glu Glu Leu Ala Gln Ala Lys Glu
                   540                 545                 550
   ccg ttt aat ctc ggg cgc tta att cgc ctc gtg aaa gaa tat cat ctg      1974
   Pro Phe Asn Leu Gly Arg Leu Ile Arg Leu Val Lys Glu Tyr His Leu
               555                 560                 565
   ctg aac ccg gtc att gtt gac tgc act tcc agc cag gca gtg gcg gat      2022
   Leu Asn Pro Val Ile Val Asp Cys Thr Ser Ser Gln Ala Val Ala Asp
           570                 575                 580
   caa tat gcc gac ttc ctg cgc gaa ggt ttc cac gtt gtc acg ccg aac      2070
   Gln Tyr Ala Asp Phe Leu Arg Glu Gly Phe His Val Val Thr Pro Asn
       585                 590                 595
   aaa aag gcc aac acc tcg tcg atg gat tac tac cat cag ttg cgt tat      2118
   Lys Lys Ala Asn Thr Ser Ser Met Asp Tyr Tyr His Gln Leu Arg Tyr
   600                 605                 610                 615
   gcg gcg gaa aaa tcg cgg cgt aaa ttc ctc tat gac acc aac gtt ggg      2166
   Ala Ala Glu Lys Ser Arg Arg Lys Phe Leu Tyr Asp Thr Asn Val Gly
                   620                 625                 630
   gct gga tta ccg gtt att gag aac ctg caa aat ctg ctc aat gca ggt      2214
   Ala Gly Leu Pro Val Ile Glu Asn Leu Gln Asn Leu Leu Asn Ala Gly
               635                 640                 645
   gat gaa ttg atg aag ttc tcc ggc att ctt tct ggt tcg ctt tct tat      2262
   Asp Glu Leu Met Lys Phe Ser Gly Ile Leu Ser Gly Ser Leu Ser Tyr
           650                 655                 660
   atc ttc ggc aag tta gac gaa ggc atg agt ttc tcc gag gcg acc acg      2310
   Ile Phe Gly Lys Leu Asp Glu Gly Met Ser Phe Ser Glu Ala Thr Thr
       665                 670                 675
   ctg gcg cgg gaa atg ggt tat acc gaa ccg gac ccg cga gat gat ctt      2358
   Leu Ala Arg Glu Met Gly Tyr Thr Glu Pro Asp Pro Arg Asp Asp Leu
   680                 685                 690                 695
   tct ggt atg gat gtg gcg cgt aaa cta ttg att ctc gct cgt gaa acg      2406
   Ser Gly Met Asp Val Ala Arg Lys Leu Leu Ile Leu Ala Arg Glu Thr
                   700                 705                 710
   gga cgt gaa ctg gag ctg gcg gat att gaa att gaa cct gtg ctg ccc      2454
   Gly Arg Glu Leu Glu Leu Ala Asp Ile Glu Ile Glu Pro Val Leu Pro
               715                 720                 725
   gca gag ttt aac gcc gag ggt gat gtt gcc gct ttt atg gcg aat ctg      2502
   Ala Glu Phe Asn Ala Glu Gly Asp Val Ala Ala Phe Met Ala Asn Leu
           730                 735                 740
   tca caa ctc gac gat ctc ttt gcc gcg cgc gtg gcg aag gcc cgt gat      2550
   Ser Gln Leu Asp Asp Leu Phe Ala Ala Arg Val Ala Lys Ala Arg Asp
       745                 750                 755
   gaa gga aaa gtt ttg cgc tat gtt ggc aat att gat gaa gat ggc gtc      2598
   Glu Gly Lys Val Leu Arg Tyr Val Gly Asn Ile Asp Glu Asp Gly Val
   760                 765                 770                 775
   tgc cgc gtg aag att gcc gaa gtg gat ggt aat gat ccg ctg ttc aaa      2646
   Cys Arg Val Lys Ile Ala Glu Val Asp Gly Asn Asp Pro Leu Phe Lys
                   780                 785                 790
   gtg aaa aat ggc gaa aac gcc ctg gcc ttc tat agc cac tat tat cag      2694
   Val Lys Asn Gly Glu Asn Ala Leu Ala Phe Tyr Ser His Tyr Tyr Gln
               795                 800                 805
   ccg ctg ccg ttg gta ctg cgc gga tat ggt gcg ggc aat gac gtt aca      2742
   Pro Leu Pro Leu Val Leu Arg Gly Tyr Gly Ala Gly Asn Asp Val Thr
           810                 815                 820
   gct gcc ggt gtc ttt gct gat ctg cta cgt acc ctc tca tgg aag tta      2790
   Ala Ala Gly Val Phe Ala Asp Leu Leu Arg Thr Leu Ser Trp Lys Leu
       825                 830                 835
   gga gtc tga c atg gtt aaa gtt tat gcc ccg gct tcc agt gcc aat atg    2839
   Gly Val        Met Val Lys Val Tyr Ala Pro Ala Ser Ser Ala Asn Met
   840                         845                 850
   agc gtc ggg ttt gat gtg ctc ggg gcg gcg gtg aca cct gtt gat ggt      2887
   Ser Val Gly Phe Asp Val Leu Gly Ala Ala Val Thr Pro Val Asp Gly
   855                 860                 865                 870
   gca ttg ctc gga gat gta gtc acg gtt gag gcg gca gag aca ttc agt      2935
   Ala Leu Leu Gly Asp Val Val Thr Val Glu Ala Ala Glu Thr Phe Ser
                   875                 880                 885
   ctc aac aac ctc gga cgc ttt gcc gat aag ctg ccg tca gaa cca cgg      2983
   Leu Asn Asn Leu Gly Arg Phe Ala Asp Lys Leu Pro Ser Glu Pro Arg
               890                 895                 900
   gaa aat atc gtt tat cag tgc tgg gag cgt ttt tgc cag gaa ctg ggt      3031
   Glu Asn Ile Val Tyr Gln Cys Trp Glu Arg Phe Cys Gln Glu Leu Gly
           905                 910                 915
   aag caa att cca gtg gcg atg acc ctg gaa aag aat atg ccg atc ggt      3079
   Lys Gln Ile Pro Val Ala Met Thr Leu Glu Lys Asn Met Pro Ile Gly
       920                 925                 930
   tcg ggc tta ggc tcc agt gcc tgt tcg gtg gtc gcg gcg ctg atg gcg      3127
   Ser Gly Leu Gly Ser Ser Ala Cys Ser Val Val Ala Ala Leu Met Ala
   935                 940                 945                 950
   atg aat gaa cac tgc ggc aag ccg ctt aat gac act cgt ttg ctg gct      3175
   Met Asn Glu His Cys Gly Lys Pro Leu Asn Asp Thr Arg Leu Leu Ala
                   955                 960                 965
   ttg atg ggc gag ctg gaa ggc cgt atc tcc ggc agc att cat tac gac      3223
   Leu Met Gly Glu Leu Glu Gly Arg Ile Ser Gly Ser Ile His Tyr Asp
               970                 975                 980
   aac gtg gca ccg tgt ttt ctc ggt ggt atg cag ttg atg atc gaa gaa      3271
   Asn Val Ala Pro Cys Phe Leu Gly Gly Met Gln Leu Met Ile Glu Glu
           985                 990                 995
   aac gac  atc atc agc cag caa  gtg cca ggg ttt gat  gag tgg ctg       3316
   Asn Asp  Ile Ile Ser Gln Gln  Val Pro Gly Phe Asp  Glu Trp Leu
       1000                 1005                 1010
   tgg gtg  ctg gcg tat ccg ggg  att aaa gtc tcg acg  gca gaa gcc       3361
   Trp Val Leu Ala Tyr Pro Gly Ile Lys Val Ser Thr Ala Glu Ala
   1015 1020 1025
   agg gct att tta ccg gcg cag tat cgc cgc cag gat tgc att gcg 3406
   Arg Ala Ile Leu Pro Ala Gln Tyr Arg Arg Gln Asp Cys Ile Ala
   1030 1035 1040
   cac ggg cga cat ctg gca ggc ttc att cac gcc tgc tat tcc cgt 3451
   His Gly Arg His Leu Ala Gly Phe Ile His Ala Cys Tyr Ser Arg
   1045 1050 1055
   cag cct gag ctt gcc gcg aag ctg atg aaa gat gtt atc gct gaa 3496
   Gln Pro Glu Leu Ala Ala Lys Leu Met Lys Asp Val Ile Ala Glu
   1060 1065 1070
   ccc tac cgt gaa cgg tta ctg cca ggc ttc cgg cag gcg cgg cag 3541
   Pro Tyr Arg Glu Arg Leu Leu Pro Gly Phe Arg Gln Ala Arg Gln
   1075 1080 1085
   gcg gtc gcg gaa atc ggc gcg gta gcg agc ggt atc tcc ggc tcc 3586
   Ala Val Ala Glu Ile Gly Ala Val Ala Ser Gly Ile Ser Gly Ser
   1090 1095 1100
   ggc ccg acc ttg ttc gct ctg tgt gac aag ccg gaa acc gcc cag 3631
   Gly Pro Thr Leu Phe Ala Leu Cys Asp Lys Pro Glu Thr Ala Gln
   1105 1110 1115
   cgc gtt gcc gac tgg ttg ggt aag aac tac ctg caa aat cag gaa 3676
   Arg Val Ala Asp Trp Leu Gly Lys Asn Tyr Leu Gln Asn Gln Glu
   1120 1125 1130
   ggt ttt gtt cat att tgc cgg ctg gat acg gcg ggc gca cga gta 3721
   Gly Phe Val His Ile Cys Arg Leu Asp Thr Ala Gly Ala Arg Val
   1135 1140 1145
   ctg gaa aac taa atg aaa ctc tac aat ctg aaa gat cac aac gag 3766
   Leu Glu Asn Met Lys Leu Tyr Asn Leu Lys Asp His Asn Glu
   1150 1155 1160
   cag gtc agc ttt gcg caa gcc gta acc cag ggg ttg ggc aaa aat 3811
   Gln Val Ser Phe Ala Gln Ala Val Thr Gln Gly Leu Gly Lys Asn
   1165 1170 1175
   cag ggg ctg ttt ttt ccg cac gac ctg ccg gaa ttc agc ctg act 3856
   Gln Gly Leu Phe Phe Pro His Asp Leu Pro Glu Phe Ser Leu Thr
   1180 1185 1190
   gaa att gat gag atg ctg aag ctg gat ttt gtc acc cgc agt gcg 3901
   Glu Ile Asp Glu Met Leu Lys Leu Asp Phe Val Thr Arg Ser Ala
   1195 1200 1205
   aag atc ctc tcg gcg ttt att ggt gat gaa atc cca cag gaa atc 3946
   Lys Ile Leu Ser Ala Phe Ile Gly Asp Glu Ile Pro Gln Glu Ile
   1210 1215 1220
   ctg gaa gag  cgc gtg cgc gcg gcg  ttt gcc ttc ccg gct  ccg gtc      3991
   Leu Glu Glu  Arg Val Arg Ala Ala  Phe Ala Phe Pro Ala  Pro Val
           1225                 1230                 1235
   gcc aat gtt  gaa agc gat gtc ggt  tgt ctg gaa ttg ttc  cac ggg      4036
   Ala Asn Val  Glu Ser Asp Val Gly  Cys Leu Glu Leu Phe  His Gly
           1240                 1245                 1250
   cca acg ctg  gca ttt aaa gat ttc  ggc ggt cgc ttt atg  gca caa      4081
   Pro Thr Leu  Ala Phe Lys Asp Phe  Gly Gly Arg Phe Met  Ala Gln
           1255                 1260                 1265
   atg ctg acc  cat att gcg ggt gat  aag cca gtg acc att  ctg acc      4126
   Met Leu Thr  His Ile Ala Gly Asp  Lys Pro Val Thr Ile  Leu Thr
           1270                 1275                 1280
   gcg acc tcc  ggt gat acc gga gcg  gca gtg gct cat gct  ttc tac      4171
   Ala Thr Ser  Gly Asp Thr Gly Ala  Ala Val Ala His Ala  Phe Tyr
           1285                 1290                 1295
   ggt tta ccg  aat gtg aaa gtg gtt  atc ctc tat cca cga  ggc aaa      4216
   Gly Leu Pro  Asn Val Lys Val Val  Ile Leu Tyr Pro Arg  Gly Lys
           1300                 1305                 1310
   atc agt cca  ctg caa gaa aaa ctg  ttc tgt aca ttg ggc  ggc aat      4261
   Ile Ser Pro  Leu Gln Glu Lys Leu  Phe Cys Thr Leu Gly  Gly Asn
           1315                 1320                 1325
   atc gaa act  gtt gcc atc gac ggc  gat ttc gat gcc tgt  cag gcg      4306
   Ile Glu Thr  Val Ala Ile Asp Gly  Asp Phe Asp Ala Cys  Gln Ala
           1330                 1335                 1340
   ctg gtg aag  cag gcg ttt gat gat  gaa gaa ctg aaa gtg  gcg cta      4351
   Leu Val Lys  Gln Ala Phe Asp Asp  Glu Glu Leu Lys Val  Ala Leu
           1345                 1350                 1355
   ggg tta aac  tcg gct aac tcg att  aac atc agc cgt ttg  ctg gcg      4396
   Gly Leu Asn  Ser Ala Asn Ser Ile  Asn Ile Ser Arg Leu  Leu Ala
           1360                 1365                 1370
   cag att tgc  tac tac ttt gaa gct  gtt gcg cag ctg ccg  cag gag      4441
   Gln Ile Cys  Tyr Tyr Phe Glu Ala  Val Ala Gln Leu Pro  Gln Glu
           1375                 1380                 1385
   acg cgc aac  cag ctg gtt gtc tcg  gtg cca agc gga aac  ttc ggc      4486
   Thr Arg Asn  Gln Leu Val Val Ser  Val Pro Ser Gly Asn  Phe Gly
           1390                 1395                 1400
   gat ttg acg  gcg ggt ctg ctg gcg  aag tca ctc ggt ctg  ccg gtg      4531
   Asp Leu Thr  Ala Gly Leu Leu Ala  Lys Ser Leu Gly Leu  Pro Val
           1405                 1410                 1415
   aaa cgt ttt  att gct gcg acc aac  gtg aac gat acc gtg  cca cgt      4576
   Lys Arg Phe  Ile Ala Ala Thr Asn  Val Asn Asp Thr Val  Pro Arg
           1420                 1425                 1430
   ttc ctg cac  gac ggt cag tgg tca  ccc aaa gcg act cag  gcg acg    4621
   Phe Leu His  Asp Gly Gln Trp Ser  Pro Lys Ala Thr Gln  Ala Thr
           1435                 1440                 1445
   tta tcc aac  gcg atg gac gtg agt  cag ccg aac aac tgg  ccg cgt    4666
   Leu Ser Asn  Ala Met Asp Val Ser  Gln Pro Asn Asn Trp  Pro Arg
           1450                 1455                 1460
   gtg gaa gag  ttg ttc cgc cgc aaa  atc tgg caa ctg aaa  gag ctg    4711
   Val Glu Glu  Leu Phe Arg Arg Lys  Ile Trp Gln Leu Lys  Glu Leu
           1465                 1470                 1475
   ggt tat gca  gcc gtg gat gat gaa  acc acg caa cag aca  atg cgt    4756
   Gly Tyr Ala  Ala Val Asp Asp Glu  Thr Thr Gln Gln Thr  Met Arg
           1480                 1485                 1490
   gag tta aaa  gaa ctg ggc tac act  tcg gag ccg cac gct  gcc gta    4801
   Glu Leu Lys  Glu Leu Gly Tyr Thr  Ser Glu Pro His Ala  Ala Val
           1495                 1500                 1505
   gct tat cgt  gcg ctg cgt gat cag  ttg aat cca ggc gaa  tat ggc    4846
   Ala Tyr Arg  Ala Leu Arg Asp Gln  Leu Asn Pro Gly Glu  Tyr Gly
           1510                 1515                 1520
   ttg ttc ctc  ggc acc gcg cat ccg  gcg aaa ttt aaa gag  agc gtg    4891
   Leu Phe Leu  Gly Thr Ala His Pro  Ala Lys Phe Lys Glu  Ser Val
           1525                 1530                 1535
   gaa gcg att  ctc ggt gaa acg ttg  gat ctg cca aaa gag  ctg gca    4936
   Glu Ala Ile  Leu Gly Glu Thr Leu  Asp Leu Pro Lys Glu  Leu Ala
           1540                 1545                 1550
   gaa cgt gct  gat tta ccc ttg ctt  tca cat aat ctg ccc  gcc gat    4981
   Glu Arg Ala  Asp Leu Pro Leu Leu  Ser His Asn Leu Pro  Ala Asp
           1555                 1560                 1565
   ttt gct gcg  ttg cgt aaa ttg atg  atg aat cat cag taa aatctattca  5030
   Phe Ala Ala  Leu Arg Lys Leu Met  Met Asn His Gln
           1570                 1575
   ttatctcaat                                                        5040
<210> 2
   <211> 21
   <212> PRT
   <213> Escherichia coli
<400> 2
<210> 3
   <211> 820
   <212> PRT
   <213> Escherichia coli
<400> 3
   Met Arg Val Leu Lys Phe Gly Gly Thr Ser Val Ala Asn Ala Glu Arg
   1 5 10 15
   Phe Leu Arg Val Ala Asp Ile Leu Glu Ser Asn Ala Arg Gln Gly Gln
   20 25 30
   Val Ala Thr Val Leu Ser Ala Pro Ala Lys Ile Thr Asn His Leu Val
   35 40 45
   Ala Met Ile Glu Lys Thr Ile Ser Gly Gln Asp Ala Leu Pro Asn Ile
   50 55 60
   Ser Asp Ala Glu Arg Ile Phe Ala Glu Leu Leu Thr Gly Leu Ala Ala
   65 70 75 80
   Ala Gln Pro Gly Phe Pro Leu Ala Gln Leu Lys Thr Phe Val Asp Gln
   85 90 95
   Glu Phe Ala Gln Ile Lys His Val Leu His Gly Ile Ser Leu Leu Gly
   100 105 110
   Gln Cys Pro Asp Ser Ile Asn Ala Ala Leu Ile Cys Arg Gly Glu Lys
   115 120 125
   Met Ser Ile Ala Ile Met Ala Gly Val Leu Glu Ala Arg Gly His Asn
   130 135 140
   Val Thr Val Ile Asp Pro Val Glu Lys Leu Leu Ala Val Gly His Tyr
   145 150 155 160
   Leu Glu Ser Thr Val Asp Ile Ala Glu Ser Thr Arg Arg Ile Ala Ala
   165 170 175
   Ser Arg Ile Pro Ala Asp His Met Val Leu Met Ala Gly Phe Thr Ala
   180 185 190
   Gly Asn Glu Lys Gly Glu Leu Val Val Leu Gly Arg Asn Gly Ser Asp
   195 200 205
   Tyr Ser Ala Ala Val Leu Ala Ala Cys Leu Arg Ala Asp Cys Cys Glu
   210 215 220
   Ile Trp Thr Asp Val Asp Gly Val Tyr Thr Cys Asp Pro Arg Gln Val
   225 230 235 240
   Pro Asp Ala Arg Leu Leu Lys Ser Met Ser Tyr Gln Glu Ala Met Glu
   245 250 255
   Leu Ser Tyr Phe Gly Ala Lys Val Leu His Pro Arg Thr Ile Thr Pro
   260 265 270
   Ile Ala Gln Phe Gln Ile Pro Cys Leu Ile Lys Asn Thr Gly Asn Pro
   275 280 285
   Gln Ala Pro Gly Thr Leu Ile Gly Ala Ser Arg Asp Glu Asp Glu Leu
   290 295 300
   Pro Val Lys Gly Ile Ser Asn Leu Asn Asn Met Ala Met Phe Ser Val
   305 310 315 320
   Ser Gly Pro Gly Met Lys Gly Met Val Gly Met Ala Ala Arg Val Phe
   325 330 335
   Ala Ala Met Ser Arg Ala Arg Ile Ser Val Val Leu Ile Thr Gln Ser
   340 345 350
   Ser Ser Glu Tyr Ser Ile Ser Phe Cys Val Pro Gln Ser Asp Cys Val
   355 360 365
   Arg Ala Glu Arg Ala Met Gln Glu Glu Phe Tyr Leu Glu Leu Lys Glu
   370 375 380
   Gly Leu Leu Glu Pro Leu Ala Val Thr Glu Arg Leu Ala Ile Ile Ser
   385 390 395 400
   Val Val Gly Asp Gly Met Arg Thr Leu Arg Gly Ile Ser Ala Lys Phe
   405 410 415
   Phe Ala Ala Leu Ala Arg Ala Asn Ile Asn Ile Val Ala Ile Ala Gln
   420 425 430
   Gly Ser Ser Glu Arg Ser Ile Ser Val Val Val Asn Asn Asp Asp Ala
   435 440 445
   Thr Thr Gly Val Arg Val Thr His Gln Met Leu Phe Asn Thr Asp Gln
   450 455 460
   Val Ile Glu Val Phe Val Ile Gly Val Gly Gly Val Gly Gly Ala Leu
   465 470 475 480
   Leu Glu Gln Leu Lys Arg Gln Gln Ser Trp Leu Lys Asn Lys His Ile
   485 490 495
   Asp Leu Arg Val Cys Gly Val Ala Asn Ser Lys Ala Leu Leu Thr Asn
   500 505 510
   Val His Gly Leu Asn Leu Glu Asn Trp Gln Glu Glu Leu Ala Gln Ala
   515 520 525
   Lys Glu Pro Phe Asn Leu Gly Arg Leu Ile Arg Leu Val Lys Glu Tyr
   530 535 540
   His Leu Leu Asn Pro Val Ile Val Asp Cys Thr Ser Ser Gln Ala Val
   545 550 555 560
   Ala Asp Gln Tyr Ala Asp Phe Leu Arg Glu Gly Phe His Val Val Thr
   565 570 575
   Pro Asn Lys Lys Ala Asn Thr Ser Ser Met Asp Tyr Tyr His Gln Leu
   580 585 590
   Arg Tyr Ala Ala Glu Lys Ser Arg Arg Lys Phe Leu Tyr Asp Thr Asn
   595 600 605
   Val Gly Ala Gly Leu Pro Val Ile Glu Asn Leu Gln Asn Leu Leu Asn
   610 615 620
   Ala Gly Asp Glu Leu Met Lys Phe Ser Gly Ile Leu Ser Gly Ser Leu
   625 630 635 640
   Ser Tyr Ile Phe Gly Lys Leu Asp Glu Gly Met Ser Phe Ser Glu Ala
   645 650 655
   Thr Thr Leu Ala Arg Glu Met Gly Tyr Thr Glu Pro Asp Pro Arg Asp
   660 665 670
   Asp Leu Ser Gly Met Asp Val Ala Arg Lys Leu Leu Ile Leu Ala Arg
   675 680 685
   Glu Thr Gly Arg Glu Leu Glu Leu Ala Asp Ile Glu Ile Glu Pro Val
   690 695 700
   Leu Pro Ala Glu Phe Asn Ala Glu Gly Asp Val Ala Ala Phe Met Ala
   705 710 715 720
   Asn Leu Ser Gln Leu Asp Asp Leu Phe Ala Ala Arg Val Ala Lys Ala
   725 730 735
   Arg Asp Glu Gly Lys Val Leu Arg Tyr Val Gly Asn Ile Asp Glu Asp
   740 745 750
   Gly Val Cys Arg Val Lys Ile Ala Glu Val Asp Gly Asn Asp Pro Leu
   755 760 765
   Phe Lys Val Lys Asn Gly Glu Asn Ala Leu Ala Phe Tyr Ser His Tyr
   770 775 780
   Tyr Gln Pro Leu Pro Leu Val Leu Arg Gly Tyr Gly Ala Gly Asn Asp
   785 790 795 800
   Val Thr Ala Ala Gly Val Phe Ala Asp Leu Leu Arg Thr Leu Ser Trp
   805 810 815
   Lys Leu Gly Val
   820
<210> 4
   <211> 310
   <212> PRT
   <213> Escherichia coli
<400> 4
   Met Val Lys Val Tyr Ala Pro Ala Ser Ser Ala Asn Met Ser Val Gly
   1               5                   10                  15
   Phe Asp Val Leu Gly Ala Ala Val Thr Pro Val Asp Gly Ala Leu Leu
               20                  25                  30
   Gly Asp Val Val Thr Val Glu Ala Ala Glu Thr Phe Ser Leu Asn Asn
           35                  40                  45
   Leu Gly Arg Phe Ala Asp Lys Leu Pro Ser Glu Pro Arg Glu Asn Ile
       50                  55                  60
   Val Tyr Gln Cys Trp Glu Arg Phe Cys Gln Glu Leu Gly Lys Gln Ile
   65                  70                  75                  80
   Pro Val Ala Met Thr Leu Glu Lys Asn Met Pro Ile Gly Ser Gly Leu
                   85                  90                  95
   Gly Ser Ser Ala Cys Ser Val Val Ala Ala Leu Met Ala Met Asn Glu
               100                 105                 110
   His Cys Gly Lys Pro Leu Asn Asp Thr Arg Leu Leu Ala Leu Met Gly
           115                 120                 125
   Glu Leu Glu Gly Arg Ile Ser Gly Ser Ile His Tyr Asp Asn Val Ala
       130                 135                 140
   Pro Cys Phe Leu Gly Gly Met Gln Leu Met Ile Glu Glu Asn Asp Ile
   145                 150                 155                 160
   Ile Ser Gln Gln Val Pro Gly Phe Asp Glu Trp Leu Trp Val Leu Ala
                   165                 170                 175
   Tyr Pro Gly Ile Lys Val Ser Thr Ala Glu Ala Arg Ala Ile Leu Pro
               180                 185                 190
   Ala Gln Tyr Arg Arg Gln Asp Cys Ile Ala His Gly Arg His Leu Ala
           195                 200                 205
   Gly Phe Ile His Ala Cys Tyr Ser Arg Gln Pro Glu Leu Ala Ala Lys
       210                 215                 220
   Leu Met Lys Asp Val Ile Ala Glu Pro Tyr Arg Glu Arg Leu Leu Pro
   225                 230                 235                 240
   Gly Phe Arg Gln Ala Arg Gln Ala Val Ala Glu Ile Gly Ala Val Ala
   245 250 255
   Ser Gly Ile Ser Gly Ser Gly Pro Thr Leu Phe Ala Leu Cys Asp Lys
   260 265 270
   Pro Glu Thr Ala Gln Arg Val Ala Asp Trp Leu Gly Lys Asn Tyr Leu
   275 280 285
   Gln Asn Gln Glu Gly Phe Val His Ile Cys Arg Leu Asp Thr Ala Gly
   290 295 300
   Ala Arg Val Leu Glu Asn
   305 310
<210> 5
   <211> 428
   <212> PRT
   <213> Escherichia coli
<400> 5 <210> 6

   <211> 66
   <212> DNA
   <213> Escherichia coli
<220>
   <221>
   <222> (1)..(66)
   <223> leader sequence
<400> 6

## Description

### Technical Field

The present invention relates to a technique for use in the fermentation industry. More specifically, the present invention relates to a method for efficiently producing L-threonine by fermentation utilizing an *Escherichia* bacterium. L-Threonine is one of the essential amino acids and is useful as an ingredient in nutritional mixtures for medical purposes. It is further utilized in various ways as an animal feed additive, and as a reagent in the pharmaceutical and chemical industries.

### Background Art

L-amino acids such as L-threonine and L-isoleucine are industrially produced by fermentation using amino acid-producing bacteria, such as coryneform bacteria or *Escherichia* bacteria which have an ability to produce these L-amino acids. As these amino acid-producing bacteria, bacterial strains isolated from nature or artificial mutants of these bacterial strains are used. Recombinants of bacterial strains in which L-amino acid biosynthesis enzymes are enhanced by gene recombination and so forth are used in order to improve the productivity.

Specifically, mutant strains of L-threonine producing *Escherichia* bacteria are known, such as a 6-dimethylaminopurine resistant strain (Japanese Patent Laid-open (Kokai) No. 5-304969) and a borrelidin resistant strain (International Patent Publication WO98/04715). Methods of using recombinant *Escherichia* bacteria to produce L-threonine are known, specifically using a strain in which the threonine operon is amplified by using a plasmid (US5,175,107), or in which the phosphoenolpyruvate carboxylase gene and the aspartase gene are amplified by using a plasmid (U.S. Patent Application No. 2002/0110876).

As genes coding for enzymes involved in the biosynthesis of L-threonine in *Escherichia coli*, the aspartokinase III gene (lysC), aspartate semialdehyde dehydrogenase gene (asd), aspartokinase I-homoserine dehydrogenase gene (thrA), homoserine kinase gene (thrB) and threonine synthase gene (thrC) and so forth are known. The thrA, thrB and thrC (thrABC) constitute the threonine operon. The threonine operon forms an attenuator structure, and the expression thereof is inhibited by isoleucine and threonine in a culture medium. It is known that the fermentation yield is improved when the leader sequence of this attenuation region or the attenuator is removed from the threonine operon (U.S. Patent No. 5,538,873, Biotechnology Letters, Vol. 24, No. 21, November 2002, and International Patent Publication WO05/049808).

To date, methods for producing L-threonine which have been developed include use of a batch culture using a fermenter initially containing all the nutrients, a fed-batch culture using a fermenter initially containing predetermined nutrients and continuously fed with one or more additional nutrients (U.S. Patent No. 5,538,873and European Patent No. 593792), and a method of regulating the saccharide concentration so that it is maintained at a predetermined level or lower (International Patent Publication WO05/014840 and International Patent Publication WO05/014843). Furthermore, another method for producing L-threonine which has been developed is a method of feeding a culture medium, or adding nutrients to a medium, so that phosphoric acid and carbon sources serve as growth limiting factors (U.S. Patent No. 5,763,230).

U.S. Patent Application No. US 2005/0025878 Al discloses a process for granulation of an animal feedstuff additive such as a fermentation product comprising amino acids or vitamins.

Sulfur is an essential factor for bacterial cell growth and is usually included in the medium for L-threonine fermentation in the form of ammonium sulfate. With regard to L-threonine production by fermentation; however, regulation of the sulfur concentration in the fermentation medium and the effect of lowering the sulfur concentration are unknown.

### Disclosure of the Invention

An object of the present invention is to provide a method for efficiently producing L-threonine by using an *Escherichia* bacterium having an ability to produce L-threonine.

The inventors of the present invention assiduously studied in order to achieve the foregoing object. As a result, the inventors found that the sulfur concentration in the medium could affect fermentation results, and that the yield of L-threonine during fermentation can be improved by regulating the sulfur concentration in the fermentation medium so that it is maintained at a predetermined level or lower, and thus accomplished the present invention.

It is an object of the present invention to provide a method for producing L-threonine comprising culturing a microorganism belonging to the genus *Escherichia* that has an ability to produce L-threonine, in a fermentation medium containing a carbon source, a nitrogen source, and a sulfur source, and collecting L-threonine from the culture, wherein the sulfur concentration in the medium is regulated so that it is 0.35 g/L or lower.

It is a further object of the present invention to provide the method as described above, wherein the microorganism is *Escherichia coli.*

It is a further object of the present invention to provide the method as described above, wherein an L-threonine biosynthesis enzyme in the microorganism is modified so that the enzyme is not subject to feedback inhibition by L-threonine.

It is a further object of the present invention to provide the method as described above, wherein the L-threonine biosynthesis enzyme is selected from the group consisting of aspartokinase, homoserine kinase, threonine synthase, and combinations thereof.

It is a further object of the present invention to provide the method as described above, wherein the sulfur source is selected from the roup consisting of sulfates, thiosulfates, sulfites, cysteine, cystine, glutathione, and combinations thereof.

It is a further object of the present invention to provide the method as described above, wherein the culture method is selected from the group consisting of a batch culture method, a fed-batch culture method, and a continuous culture method.

It is a further object of the present invention to provide the method as described above, wherein the culture method is a fed-batch culture method or a continuous culture method, wherein a feed medium containing a sulfur source is added to the culture in a fermenter.

It is a further object of the present invention to provide the method as described above, wherein said feed medium further contains a carbon source and a nutrient having a growth promoting effect, and wherein said feed medium is added to the fermenter continuously or intermittently so that concentration of the carbon source in the culture medium is maintained at 30 g/L or lower after the end of the logarithmic growth of the microorganism.

It is a further object of the present invention to provide a method for producing animal feed additive based on fermentation broth comprising:
A) culturing a microorganism belonging to the genus *Escherichia* that has an ability to produce L-threonine, in a fermentation medium containing a carbon source, a nitrogen source, and a sulfur source,
B) conducting the fermentation wherein the sulfur concentration in the medium is regulated so that it is 0.35 g/L or lower,
C) drying the crude fermentation broth to a water content of 10% or less by weight.

### Brief Description of the Drawing

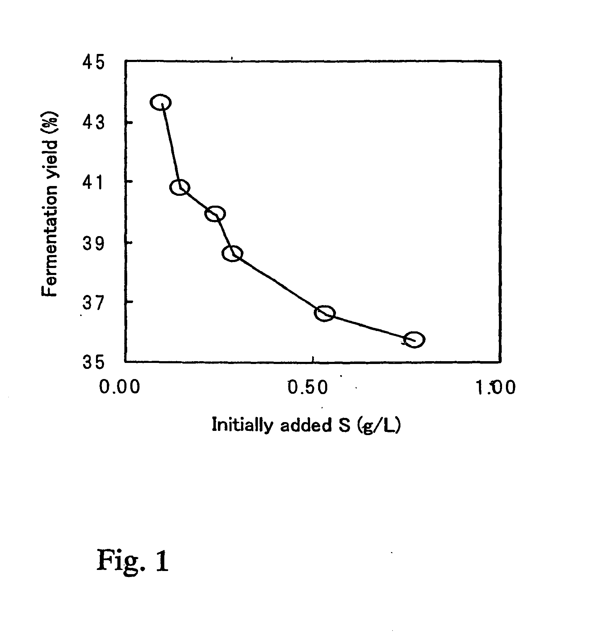

Fig. 1 shows the relationship between the amount of sulfur initially added to a medium, and the L-threonine yield.

### Detailed Description of the Preferred Embodiments

### <1> The method of the present invention

The method of the present invention is a method for producing L-threonine by culturing a microorganism belonging to the genus *Escherichia* that has an ability to produce L-threonine, in a fermentation medium containing a carbon source, a nitrogen source, and a sulfur source so that L-threonine is produced in the medium, wherein the sulfur concentration in the medium is regulated so that it is maintained at a predetermined level or lower. In the present invention, the term "sulfur concentration" means the concentration of the sulfur source in terms of sulfur atom concentration.

The medium used for the present invention may be any medium so long as it is a liquid medium which contains a carbon source, a nitrogen source, and a sulfur source as nutrients, and the medium is not particularly limited except that it is adjusted so that the sulfur concentration is maintained at a predetermined level or lower. The sulfur source may be any sulfur-containing source. Salts of sulfuric acid, such as sulfates, thiosulfates, and sulfites, as well as sulfur-containing amino acids such as cysteine, cystine, and glutathione are desirable. Among these, ammonium sulfate is particularly preferable. The salts are not particularly limited, and ammonium salts, calcium salts,sodium salts, potassium salts, magnesium salts, manganese salts, and iron salts may be used. Furthermore, the medium may contain only one type, or two or more types, of these substances. The concentration indicated by the phrase "predetermined level or lower" is 0.35 g/L or lower, preferably 0.25 g/L or lower, particularly preferably 0.10 g/L or lower of sulfur in the fermentation medium. One of the characteristics of the present invention is that the sulfur concentration in the fermentation medium is regulated. For the method of the present invention, a batch culture, fed-batch culture, and/or a continuous culture can be employed, and the sulfur concentration in the medium may be regulated to be at a predetermined level or lower in the initial medium, or limited to be at a predetermined level or lower by controlling the sulfur concentration in the feed medium or by using these techniques in combination. The same sulfur source may be used for the initial medium and the feed medium, or the sulfur source in the feed medium may be different from that used in the initial medium.

In the present invention, a fed-batch culture refers to a culture method wherein the medium is added, continuously or intermittently, into a vessel during the culture, and not withdrawing the medium from the vessel before completion of culture. The continuous culture refers to a method of continuously or intermittently adding a medium into a vessel during the culture, and extracting the medium from the vessel (normally, in an amount equivalent to the medium-fed). The term "initial medium" means a medium used for batch culture before the feed medium is added in the fed-batch culture or continuous culture, and the term "feed medium" means a medium to be supplied to a fermenter when fed-batch culture or continuous culture is performed. The feed medium may contain all or a part of the components necessary for the growth of a microorganism. In the present invention, the term "fermentation medium" means a medium contained in a fermenter, and L-threonine is collected from this fermentation medium. Furthermore, in the present invention, the term "fermenter" means a vessel in which L-threonine fermentation is performed, and the shape thereof is not limited. A fermentation tank or a jar fermenter may be used. Furthermore, the volume of the fermenter is not limited so long as L-threonine can be produced and collected.

Although the sulfur concentration is preferably limited to a predetermined level or lower throughout the whole culture process, it may be limited during just a part of the process. For example, when the method of the present invention includes a stage at which cells proliferate (growth phase) and a stage at which L-threonine is produced (L-threonine production phase), it is sufficient that the sulfur concentration is limited to a predetermined level or lower in the L-threonine production phase. In the growth phase (during cell proliferation), sulfur may be present in the medium at more than the predetermined amount, or the sulfur concentration may be limited to the predetermined level or lower. Furthermore, during the stage at which L-threonine is produced, the sulfur content does not need to be in the aforementioned range throughout the whole period of this stage, and the sulfur content may be at the aforementioned level or higher during an early period of that stage, and may be reduced with the culture time. Furthermore, sulfur may be added intermittently when it runs short. The term "growth phase" used in the present invention means a period within 3 hours, preferably 6 hours, particularly preferably 10 hours, from the start of the culture, during which period the carbon source is primarily consumed for the bacterial cell growth, that is, cells logarithmically proliferate. The term "L-threonine production phase" used in the present invention means a period after the initial 3 hours, preferably 6 hours, particularly preferably 10 hours, from the start of culture, during which the carbon source is primarily consumed for L-threonine production.

It is sufficient that the fermentation medium contains a minimum amount of sulfur which is required for the growth of the microorganism; however, the amount of sulfur may temporarily run short. The phrase "run(s) short" means that the sulfur concentration is reduced compared to earlier points in the culture, and may even become "0". The term "temporarily" means that, for example, sulfur may run short for a period corresponding to about 20%, about 40%, or about 60% at most, of the whole fermentation period. During the period when sulfur runs short, although the concentration may temporarily be 0, sulfur is desirably present in the fermentation medium at a concentration of 1 µg/L or more, 10 µg/L or more, or 100 µg/L or more. Thus, even if the sulfur concentration temporarily becomes 0, a culture of a medium containing sulfur for any period is encompassed within the expression "culturing a microorganism belonging to the genus *Escherichia* in a fermentation medium containing a carbon source, a nitrogen source, and a sulfur source". The sulfur concentration in the culture medium can be measured by the ion chromatography method and the hot flask method.

Furthermore, according to the present invention, the sulfur concentration in the feed medium may be adjusted so that it is limited to a predetermined level or lower when fed-batch culture can be employed. For example, when the sulfur concentration is limited by using a fed-batch culture, it is preferable to control the sulfur concentration in the fermentation medium to be 0.35 g/L or lower, desirably 0.25 g/L or lower, more desirably 0.10 g/L or lower.

Examples of the carbon source used in the present invention include saccharides such as glucose, glycerol, fructose, sucrose, maltose, mannose, galactose, starch hydrolysate, and molasses. Glucose and sucrose are particularly preferred. In addition, organic acids such as acetic acid and citric acid, and alcohols such as ethanol can also be used alone or in combination with another carbon source. Furthermore, the raw material of the carbon source may be cane molasses, beet molasses, high test molasses and citrus molasses, and hydrolysates of natural raw materials such as cellulose, starch, corn, cereal and tapioca. Furthermore, carbon dioxide dissolved in a culture medium can also be used as a carbon source. These carbon sources can be used in the initial medium and/or in the feed medium. The medium may contain one or more types of these carbon sources. Furthermore, the same carbon source may be used for the initial medium and the feed medium, or the carbon source of the feed medium may be different from that of the initial medium. For example, glucose may be used in the initial medium, while sucrose may be used in the feed medium.

Examples of the nitrogen source used in the present invention include ammonia, ammonium salts such as ammonium sulfate, ammonium carbonate, ammonium chloride, ammonium phosphate, ammonium acetate and urea, nitrates, and so forth. Ammonia gas and aqueous ammonia which are used to adjust the pH can also be utilized as the nitrogen source. Furthermore, peptone, yeast extract, meat extract, malt extract, corn steep liquor, soybean hydrolysate, and so forth can also be utilized. The medium may contain one or more types of these nitrogen sources. These nitrogen sources can also be used in the initial medium and/or in the feed medium. Furthermore, the same nitrogen source can be used in both the initial medium and in the feed medium, and the nitrogen source of the feed medium may be different from that of the initial medium.

Furthermore, the medium of the present invention preferably contains a phosphoric acid source in addition to a carbon source, a nitrogen source, and sulfur. As the phosphoric acid source, potassium dihydrogenphosphate, dipotassium hydrogenphosphate, and phosphate polymers such as pyrophosphoric acid can be utilized.

Furthermore, the medium of the present invention may contain a growth promoting factor (a nutrient having a growth promoting effect) in addiction to a carbon source, a nitrogen source, and sulfur. Growth promoting factors which can be used include trace metals, amino acids, vitamins, fatty acids, nucleic acids, as well as peptone, casamino acid, yeast extract, soybean protein hydrolysate.

Examples of the trace metals include iron, manganese, magnesium, calcium, potassium, sodium and so forth. Examples of the vitamins include vitamin B₁, vitamin B₂, vitamin B₆, nicotinic acid, nicotinic acid amide, vitamin B₁₂, and so forth. These growth promoting factors may be contained in the initial medium or in the feed medium.

Furthermore, when an auxotrophic mutant that requires an amino acid or the like for growth thereof is used, it is preferable to add the required nutrient to the medium. In particular, since the L-threonine biosynthetic pathway is often enhanced and the L-threonine degrading ability is often attenuated in the L-threonine producing bacteria that can be used for the present invention as described below, L-lysine, L-homoserine, L-isoleucine, L-methionine, or a combination thereof are preferably added.

The initial medium and the feed medium may have the same or different compositions. Furthermore, the initial medium and the feed medium may have the same or different sulfur concentration. Furthermore, when the feed medium is added at multiple stages, the composition of the feed medium added at each stage may be the same or different.

The culture is preferably performed with aeration, preferably at a fermentation temperature of 20 to 45°C, particularly preferably at 33 to 42°C. The oxygen concentration is preferably adjusted to 5 to 30%, more preferably to about 10%. Furthermore, aeration is preferably performed with the pH adjusted to 5 to 9. If the pH is lowered during the culture, for example, calcium carbonate or an alkali such as ammonia gas and aqueous ammonia may be added to neutralize the culture. When the culture is performed under such conditions, preferably for about 10 to 120 hours, a marked amount of L-threonine accumulates in the culture medium. The concentration of accumulated L-threonine is not limited so long as L-threonine can be isolated and collected from the medium, and it is 50 g/L or higher, desirably 75 g/L or higher, more desirably 100 g/L or higher.

In the present invention, culture of the microorganism may be performed by seed culture and/or a main culture in order to induce accumulation of L-threonine higher than a certain level. The seed culture may be performed by shaking, using a flask or the like, or a batch culture. The main culture may be performed as fed-batch culture or a continuous culture. Alternatively, both the seed culture and the main culture may be performed as batch culture.

In these culture methods, when the L-threonine concentration reaches a predetermined level, some of the fermentation broth may be withdrawn, and fresh medium may be added to repeat the culture. As the fresh medium, a medium containing a carbon source and a nutrient having a growth promoting effect (growth promoting factor) is preferred, and it preferably contains sulfur at a predetermined concentration or lower. The expression "predetermined concentration or lower" means that the medium to be added is adjusted so that the sulfur concentration in the fermentation medium becomes 0.35 g/L or lower, desirably 0.25 g/L or lower, more desirably 0.10 g/L or lower. As the carbon source, glucose, sucrose, and fructose are preferred. As the growth promoting factor, nitrogen sources, phosphoric acid, amino acids and so forth are preferred. As the nitrogen source, ammonia, ammonium salts such as ammonium sulfate, ammonium carbonate, ammonium chloride, ammonium phosphate, ammonium acetate and urea, nitrates, and so forth can be used. Furthermore, as the phosphoric acid source, potassium dihydrogenphosphate and dipotassium hydrogenphosphate can be used. As for amino acids, when an auxotrophic mutant strain is used, it is preferable to supplement with the required amino acid.

When fed-batch culture or continuous culture is performed according to the present invention, the addition of the feed medium may be intermittently suspended, so that the supply of saccharide or nutrients is temporarily suspended. The addition of the feed medium is preferably suspended for, at the most, 30%, desirably 20%, particularly desirably 10%, of the feeding time. The "feeding time" means the period from beginning of the first addition of the feed medium to end of the last addition of the feed medium. The suspension of the addition of feed medium can be less than 30%, and most preferably, less than 10%. When the feed medium is intermittently added, the feed medium may be initially added over a predetermined time, and the second and following additions may be controlled so that it is started when an increase in the pH or the dissolved oxygen concentration is detected by a computer. Such detection typically occurs upon depletion of the carbon source in the fermentation medium during an addition/suspension period prior to a certain addition period, and thus the substrate concentration in the culture tank should be automatically and consistently maintained at a low level (U.S. Patent No. 5,912,113).

The feed medium used for the fed-batch culture is preferably a medium containing a carbon source and a nutrient having a growth promoting effect (growth promoting factor), and may contain sulfur so that the sulfur concentration in the fermentation medium is maintained at a predetermined concentration or lower. The expression "predetermined concentration or lower" as used herein means that the added medium is adjusted so that the sulfur concentration in the fermentation medium is maintained at a concentration of 0.35 g/L or lower, desirably 0.25 g/L or lower, more desirably 0.10 g/L or lower. Although the sulfur concentration of the feed medium itself may be within or out of the aforementioned concentration rage, it is preferably within the aforementioned concentration range.

As the carbon source, glucose, sucrose, and fructose are preferred. As the growth promoting factor, nitrogen sources, phosphoric acid, amino acids and so forth are preferred. As the nitrogen source, ammonia, ammonium salts such as ammonium sulfate, ammonium carbonate, ammonium chloride, ammonium phosphate, ammonium acetate and urea, nitrates, and so forth can be used. Furthermore, as the phosphoric acid source, potassium dihydrogenphosphate and dipotassium hydrogenphosphate can be used. As for the amino acids, when an auxotrophic mutant strain is used, it is preferable to supplement with the required amino acid. Furthermore, the feed medium may be of one type, or a mixture of two or more types of media. When two or more types of feed media are used, the media may be mixed and added by using one feed can, or by using two or more feed cans.

Furthermore, when a fed-batch culture is performed, the addition is preferably performed so that the amount of saccharide does not exceed 30 g/L, preferably 20 g/L, more preferably 10 g/L, of the whole fed-batch culture medium or fermentation medium. In particular, the saccharide concentration is preferably controlled to be within the aforementioned concentration range after the completion of the logarithmic growth of the microorganism. The feeding rate of the carbon source can be controlled by the method described in U.S. Patent No. 5,912,113. Furthermore, the saccharide and phosphoric acid are preferably fed so that the concentrations are such that the saccharide and phosphoric acid are limiting factors for the bacterial cell growth. Phosphoric acid is present in the feed medium so that the phosphorous/carbon (P/C) ratio is 2 or lower, preferably 1.5 or lower, more preferably 1 or lower (U.S. Patent No. 5,763,230).

When the continuous culture method is used for the present invention, the medium may be withdrawn and added simultaneously, or a part of the medium may be extracted, and then the medium may be added. Furthermore, the method may also be a continuous culture method of withdrawing the culture medium containing L-threonine and bacterial cells and recycling only the cells to the fermenter (French Patent No. 2669935). As the method of continuously or intermittently adding a nutrient source, the same method as used in the fed-batch culture is used.

When the culture medium is intermittently withdrawn, a part of L-threonine is collected when the L-threonine concentration reaches a predetermined level, and fresh medium is added to continue the culture. Furthermore, as for the amount of the fresh medium to be added, the final amount of the total medium in the culture after the addition of the fresh medium is equal to the amount of the culture medium before the extraction. The term "equal" as used herein means about 93 to 107% of the amount of the culture medium before the extraction.

When the culture medium is continuously withdrawn, the withdrawal is preferably started at the same time as, or after, the addition of the nutrient medium. For example, the starting time is, at maximum, 5 hours, preferably 3 hours, more preferably 1 hour, after the start of the addition. Furthermore, the withdrawal amount of the culture medium is preferably equal to the amount of the medium added.

The continuous culture method of reusing bacterial cells is a method of intermittently or continuously with drawing the fermentation medium when the amino acid concentration reaches a predetermined level, withdrawing only L-threonine, and re-circulating filtration residues containing bacterial cells in the fermenter, and it can be performed by referring to, for example, French Patent No. 2669935.

Analysis of L-threonine and other amino acids can be performed by anion exchange chromatography followed by ninhydrin derivation as described in Spackman et al.(Analytical Chemistry 30:1190-1206 (1958)) or by reversed phase HPLC as described in Lindroth et al. (Analytical Chemictry 51:1167-1174).

### <2> The method for producing animal feed additive based on fermentation broth produced by this invention

An animal feed additive based on fermentation broth produced by this invention can be prepared by following separation method.

L-threonine separation methods such as centrifuging, filtering, decanting flocculating or a combination of these can be used to remove or reduce biomass.

The broth obtained by this invention can be thickened or concentrated using known methods such as a rotary evaporator, thin layer evaporator, reverse osmosis, or nanofiltration.(FR8613346B,US4,997,754, EP410005B, JP1073646B)

The concentrated broth is then processed using the methods of freeze-drying, spray-drying, spray granulation or any other process to give a preferably free flowing, finely divided powder for animal feed additive. This free-flowing finely divided powder can be converted into a coarse-grained, very free flowing, strable and largely dust-free product by using suitable compacting or granulating processes. Altogether, more than 90% of water is removed in this way so that water concentration of the feed animal additive is less than 10%, preferably less than 5% by weight.

The protein content of the feed additive can be less than 10%, preferably less than 5% by weight, and the concentration of L-threonine can be more than 50%, preferably more than 85%, more preferably more than 95%. (US5,431,933, JP1214636B, US4,956,471, US4,777,051, US4946654, US5,840358, US6,238,714, US2005/0025878)

The separation step described above do not necessarily have to be performed, but may be combined in a technically expedient manner.

### <3> Escherichia bacteria that can be used for the present invention

*Escherichia* bacteria that can be used for the present invention are *Escherichia* bacteria having an ability to produce L-threonine, and the term "ability to produce L-threonine" used in the present invention means an ability, when the bacteria are cultured in a medium, of producing free L-threonine in the medium, that is, outside the cells, to such an extent that L-threonine can be collected from the medium. Preferably, the *Escherichia* bacterium used to the present invention is a strain which is bred so that the strain can produce a higher amount of L-threonine as compared with wild-type strains or parental strains. Specifically, it is preferable that the *Escherichia* bacterium produces an amount of L-threonine of 30 g/L or higher, more preferably 50 g/L or higher, particularly preferably 75 g/L or higher, using a typical culture method in which the sulfur concentration is not adjusted.

Parent strains of *Escherichia* bacteria used to obtain the *Escherichia* bacteria suitably used for the present invention are not particularly limited, and specific examples thereof include those mentioned in the work of Neidbardt *et al.* (Neidhardt, F.C. et al., Escherichia coli and Salmonella Typhimurium, American Society for Microbiology, Washington D.C., 1029, Table 1). Among these, for example, *Escherichia coli* is preferably used. Specific examples of the *Escherichia coli* include *Escherichia coli* W3110 (ATCC 27325) and *Escherichia coli* MG1655 (ATCC 47076), both of which are derived from a prototype wild-type strain K12, and so forth.

As for obtaining these strains, they can be obtained from, for example, American Type Culture Collection (address: P.O. Box 1549 Manassas, VA 20108, United States of America). Each bacterial strain is given a corresponding registration number, and each strain can be ordered by using this registration number. The registration numbers corresponding to bacterial strains are listed in the catalogue of American Type Culture Collection.

### <3-1> Impartation of L-threonine producing ability

Hereinafter, the method for imparting an L-threonine producing ability to an *Escherichia* bacterium will be described.

To impart an L-threonine producing ability, methods can be used which have been conventionally adopted for breeding *Escherichia* bacteria or coryneform bacteria, such as acquisition of an auxotrophic mutant, analogue resistant strain, or metabolism control mutant strain, all of which have an L-threonine producing ability, as well as construction of a recombinant strain in which an activity of an L-threonine biosynthesis enzyme is enhanced. For example, a mutant or recombinant strain can be modified so that an L-threonine biosynthesis enzyme is not subject to feedback inhibition, or a recombinant strain can be modified to enhance expression of an L-threonine biosynthesis enzyme gene. In the breeding of L-threonine producing bacteria by these methods, properties such as auxotrophy, analogue resistance, and a metabolic control mutation may be imparted either singly or in combination. When activity of an L-threonine biosynthesis enzyme is enhanced, one or more of these enzymes' activities may be enhanced. Furthermore, imparting the properties mentioned above and enhancing enzyme activity, as mentioned above, may be implemented in combination.

An example of a method for imparting L-threonine producing ability to an *Escherichia* bacterium, or enhancing the L-threonine producing ability by enhancing activity of an L-threonine biosynthesis enzyme, will be explained below. An enzymatic activity can be enhanced by, for example, introducing a mutation into a gene coding for the enzyme or amplifying the gene so that intracellular activity of the enzyme increases. These can be achieved by utilizing gene recombination techniques.

Examples of the genes which encode L-threonine biosynthesis enzymes include the aspartokinase III gene (*lysC*), aspartate semialdehyde dehydrogenase gene (*asd*), aspartokinase I gene included in the *thr* operon (*thrA*, nucleotide numbers 337 to 2799 of SEQ ID NO: 1), homoserine kinase gene (*thrB*, nucleotide numbers 2801 to 3733 of SEQ ID NO: 1), and threonine synthase gene (*thrC*, nucleotide numbers 3734 to 5020 of SEQ ID NO: 1). Shown in the parentheses are the abbreviated names of these genes. Two or more of these genes may be introduced into a bacterium. L-threonine biosynthetic genes may be introduced into an *Escherichia* bacterium in which threonine degradation is suppressed. Examples of the *Escherichia* bacterium in which threonine degradation is suppressed include the TDH6 strain, which is deficient in threonine dehydrogenase activity (EP1149911A), and so forth.

Enzymatic activities of L-threonine biosynthesis enzymes are inhibited by L-threonine as the final product. Therefore, to construct an L-threonine producing bacterium, modifying L-threonine biosynthesis system genes so that the encoded enzymes are not subject to feedback inhibition by L-threonine is beneficial. The aforementioned *thrA, thrB*, and *thrC* genes constitute the threonine operon, and the threonine operon has an attenuator structure. Expression of the threonine operon is inhibited by isoleucine and threonine present in the culture medium, and is suppressed by attenuation. The aforementioned modification can be achieved by removing the leader sequence in the attenuation region (SEQ ID NO: 6), or by removing the attenuator (SEQ ID NO: 1) (International Patent Publication WO02/26993; Biotechnology Letters, Vol. 24, No. 21, November 2002; International Patent Publication WO05/049808, WO98/04715,WO03/097839). In particular, it is desirable to modify the threonine operon so that the sequence of the nucleotides 188 to 310 is removed from the sequence of SEQ ID NO: 1. Alternatively, modifying the threonine operon so that the sequence of the nucleotides 148 to 310 is removed from the sequence of SEQ ID NO: 1 is also desirable.

A native promoter exists in an upstream region of the threonine operon, and it may be replaced with a non-native promoter (refer to WO98/04715). Alternatively, the threonine operon may be constructed so that expression of the genes involved in threonine biosynthesis is regulated by the repressor and promoter of lambda phage (refer to European Patent No. 0593792). Furthermore, an *Escherichia* bacterium modified so that it is not subject to feedback inhibition by L-threonine can also be obtained by selecting a strain resistant to α-amino-β-hydroxyvaleric acid (AHV) (refer to WO03/097839).

Furthermore, multiple copies of the threonine operon which has been modified so that it is not subject to feedback inhibition by L-threonine preferably can be present. Alternatively, the operon is ligated to a strong promoter so that expression is increased. The copy number of the L-threonine operon can be increased by amplifying it using a plasmid and/or transferring it onto a chromosome using a transposon, Mu-phage, or the like (US5,175,107).

Furthermore, an aspartokinase III gene (*lysC*) which has been modified so that it is not subject to feedback inhibition by L-lysine is preferably used. Such a *lysC* gene can be obtained by the method described in U.S. Patent No. 5,932,453.

In addition to the L-threonine biosynthesis enzymes, it is also preferable to enhance expression of genes relating to the glycolytic pathway, TCA cycle, or respiration chain, genes that regulate expression of a gene, and saccharide uptake genes. Examples of the genes that have an effect on L-threonine production include the transhydrogenase (*pntAB*) gene (European Patent No. 733712), phosphoenolpyruvate carboxylase gene (*pepC*) (International Patent Publication WO95/06114, US2005-0136518), phosphoenolpyruvate synthase gene (*pps*) (European Patent No. 877090), and pyruvate carboxylase gene of coryneform or *Bacillus* bacteria (International Patent Publication WO99/18228; European Patent Publication No. 1092776).

Furthermore, it is also preferable to enhance expression of a gene that imparts L-threonine resistance, or a gene that imparts L-homoserine resistance, or both in combination to a host. Examples of a gene that imparts resistance include the *rhtA* gene (Res. Microbiol., 2003 Mar., 154(2): 123-35), *rhtB* .gene (European Patent Publication No. 0994190), *rhtC* gene (European Patent Publication No. 1013765), *yfiK* gene, and *yeaS* gene (European Patent Publication No. 1016710). Furthermore, the methods for imparting L-threonine resistance to a host are described in European Patent Publication No. 0994190 and International Patent Publication WO90/04636.

Activities of enzymes encoded by the aforementioned genes can be increased by enhancing expression of the genes, for example, by increasing the copy numbers of the genes in the cells using gene recombination techniques. For example, a DNA fragment containing a target gene can be ligated to a vector that functions in a host microorganism, preferably a multi-copy type vector, and the host microorganism can be transformed with this vector DNA.

When an *Escherichia coli* gene is used as the target gene, it can be obtained by preparing primers by referring to the known gene sequence in *Escherichia coli* MG1655 or W3110 registered at GenBank and performing a polymerase chain reaction (PCR) using chromosomal DNA of *Escherichia coli* as a template (refer to White, T.J. et al., Trends Genet. 5, 185 (1989)). The target genes of other microorganisms can also be obtained from chromosomal DNAs or chromosomal DNA libraries of the microorganisms by PCR using primers prepared based on the known gene information for the microorganisms or sequence information from GenBank, or hybridization using an oligonucleotide prepared based on the aforementioned sequence information as a probe. Chromosomal DNA can be prepared from a donor microorganism, for example, by the method of Saito and Miura (refer to H. Saito and K. Miura, Biochem. Biophys. Acta, 72, 619 (1963); Text for Bioengineering Experiments, Edited by the Society for Bioscience and Bioengineering, Japan, pp.97-98, Baifukan, 1992), and so forth.

Furthermore, since nucleotide sequences of genes coding for a target enzyme may vary depending on the species of *Escherichia* bacteria or bacterial strains, the target gene used for the present invention is not limited to known genes or gene sequences registered at GenBank, and may be a mutant or artificially modified gene. Such a mutant or modified gene may code for a protein which has a sequence that includes substitutions, deletions, insertions, additions, or the like, of one or several amino acid residues at one or more sites, so long as the function of the encoded target protein, that is, the L-threonine production ability, can be improved by amplification of the gene. The number meant by the term "several" used herein varies depending on positions of amino acid residues in the three-dimensional structure of the protein and types of the amino acid residues. However, it is specifically 1 to 20, preferably 1 to 10, more preferably 1 to 5.

The aforementioned substitution is preferably a conservative substitution, which is a neutral mutation causing no functional change. The conservative mutation is a mutation in which substitution occurs among Phe, Trp and Tyr when the substitution site is an aromatic amino acid, among Leu, Ile and Val when the substitution site is a hydrophobic amino acid, between Gln and Asn when the substitution site is a polar amino acid, among Lys, Arg and His when the substitution site is a basic amino acid, between Asp and Glu when the substitution site is an acidic amino acid, and between Ser and Thr when the substitution site is an amino acid having hydroxyl group. More specifically, examples of the conservative substitution include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for He, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr and substitution of Met, Ile or Leu for Val.

Furthermore, a homologous gene may also be used, so long as it has the same function as the target gene. Specifically, the homologous gene should encode a protein that has a homology of 80% or more, preferably 90% or more, more preferably 95% or more, particularly preferably 97% or more, with respect to a sequence of a known protein. Furthermore, since the degeneracy of a gene varies depending on the host into which the gene is introduced, a gene with substitutions of codons that can be easily used in a host may be used. Similarly, so long as the L-threonine producing ability of the target gene can be improved by amplification of the gene, the gene may be extended or shortened at either the N-terminus or C-terminus. The length of the extension or shortening is, for example, 50 or less, preferably 20 or less, more preferably 10 or less, particularly preferably 5 or less, in terms of the number of amino acid residues. More specifically, the protein may have an amino acid sequence which is shortened by 5-50 amino acid residues at either the N-terminus and/or the C-terminus.

A gene homologous to the target gene can be obtained by modifying the nucleotide sequence by, for example, site-specific mutagenesis, so that the encoded protein includes substitutions, deletions, insertions, or additions of amino acid residues at a specific site. Furthermore, such a gene can also be obtained by known mutation treatments, as described below. Examples of the these mutation treatments include a method of treating the nucleotide sequence of the target gene in vitro with hydroxylamine or the like, a method of treating a microorganism having the gene, for example, an *Escherichia* bacterium, with UV irradiation or a typical mutagenesis agent such as N-methyl-N'-nitro-N-nitrosoguanidine (NTG) or ethyl methanesulfonate (EMS), and a method of introducing a mutation by error-prone PCR. Furthermore, the aforementioned substitutions, deletions, insertions, additions, inversions, or the like, of amino acid residues include naturally occurring mutations (mutant or variant), such as mutations based on individual differences and/or species differences of the microorganism having the target gene. Whether these genes code for a protein that improves the L-threonine producing ability when their expression levels are increased can be confirmed by, for example, introducing these genes into a wild-type strain or a strain having L-threonine producing ability of *Escherichia coli* and examining whether the L-threonine producing ability is improved.

The target gene may also be DNA that hybridizes with a target nucleotide sequence, or a complementary sequence thereof, or a probe prepared from these sequences under stringent conditions and codes for a protein that improves the L-threonine producing ability of *Escherichia* bacteria by enhancing the expression. The "stringent conditions" referred to herein include conditions under which a so-called specific hybrid is formed, and a non-specific hybrid is not formed. It is difficult to clearly express this condition by using any numerical value. However, for example, the stringent conditions include conditions under which DNAs having high homology, for example, DNAs having a homology of 50% or more, preferably 60% or more, more preferably 70% or more, more preferably 80% or more, more preferably 90% or more, further preferably 95% or more, most preferably 97% or more, hybridize with each other, but DNAs having a homology lower than the above do not hybridize with each other. Alternatively, the stringent conditions are exemplified by washing one time, preferably 2 to 3 times, under conditions for hybridization at a salt concentration which are typical for washing in Southern hybridization, i.e., 1 x SSC, 0.1% SDS at 60°C, preferably 0.1 x SSC, 0.1% SDS at 68°C.

Then, a recombinant DNA is prepared by ligating the target gene amplified by PCR to a vector DNA that can function in the cell of a host microorganism. Such a vector includes a vector autonomously replicable in the cell of the host microorganism. Examples of the vector autonomously replicable in *Escherichia coli* cells include pUC19, pUC18, pHSG299, pHSG399, pHSG398, pACYC184, (pHSG and pACYC are available from Takara Bio Inc.), RSF1010, pBR322, pMW219 (pMW is available from Nippon Gene), and so forth.

A recombinant DNA prepared as described above can be introduced into a microorganism by a known transformation method. Examples include a method of treating recipient cells with calcium chloride so as to increase the permeability of DNA, which has been reported for *Escherichia coli* K-12 (Mandel, M. and Higa, A., J. Mol. Biol., 53, 159 (1970)), a method of preparing competent cells from cells which are at the growth phase followed by introducing DNA thereinto, which has been reported for *Bacillus subtilis* (Duncan, C.H., Wilson, G.A. and Young, F.E., Gene, 1, 153 (1977)), and so forth. In addition to these, a method of making DNA-recipient cells into protoplasts or spheroplasts, which can easily take up recombinant DNA, followed by introducing the recombinant DNA into the DNA-recipient cells, which is known to be applicable to *Bacillus subtilis*, actinomycetes, and yeasts (Chang, S. and Cohen, S.N., Molec. Gen. Genet., 168, 111, 1979; Bibb, M.J., Ward, J.M. and Hopwood, O.A., Nature, 274, 398, 1978; Hinnen, A., Hicks, J.B. and Fink, G.R., Proc. Natl. Sci. USA, 75, 1929 (1978)), may be used.

Increasing the copy number of a gene can also be achieved by introducing multiple copies of the gene into the chromosomal DNA of a microorganism. In order to introduce multiple copies, homologous recombination can be carried out by using a sequence whose multiple copies exist on a chromosomal DNA as targets. As sequences whose multiple copies exist on a chromosomal DNA, repetitive DNA and inverted repeats existing at the end of a transposable element can be used. A target gene may also be introduced into an unnecessary gene on a chromosome by homologous recombination, or a target gene may be introduced into an unnecessary region in tandem. Alternatively, as disclosed in Japanese Patent Laid-open No. 2-109985, it is also possible to incorporate a target gene into a transposon, and allow it to be transferred to introduce multiple copies of the genes into a chromosomal DNA (refer to U.S. Patent No. 5,595,889). Whether the target gene has been transferred to a chromosome can be confirmed by Southern hybridization using a part of the target gene as a probe.

Furthermore, in addition to increasing the gene copy number, expression of the target gene can be enhanced by replacing an expression control sequence such as a promoter of the target gene on a chromosomal DNA or plasmid with a stronger one, amplifying a regulator that increases the expression, or deleting/attenuating a regulator that decreases the expression as described in International Patent Publication WO00/18935. For example, the lac promoter, trp promoter, trc promoter, Pr promoter derived from lambda phage, and so forth, are known as strong promoters. Furthermore, the promoter of the target gene can be modified to be stronger by introducing a nucleotide substitution into the promoter region. Evaluation of promoter potency, as well as a description of known strong promoters, are set forth in Goldstein et al. (Prokaryotic promoters in biotechnology, Biotechnol. Annu. Rev., 1995, 1, 105-128). Furthermore, it is known that substitution of a spacer between the ribosome binding site (RBS) and the start codon, in particular, substitution of several nucleotides in the sequence immediately upstream from the start codon, has a great impact on mRNA translation efficiency, and these nucleotides can be modified. An expression control region such as a promoter can also be determined by using a promoter search vector or a gene analysis software program such as GENETYX. Expression of the target gene is enhanced by substitution or modification of these promoters. The expression control sequence can be substituted by using, for example, a temperature-sensitive plasmid.

As a result of the increase in activity by such methods, the enzymatic activity increases by at least 10%, 25%, 50%, 100%, 200%, 400%, 600%, or 1000% as compared with a wild-type strain or unmodified strain. Furthermore, these techniques for increasing enzymatic activity can be implemented in combination with each other or in combination with a decrease of an enzymatic activity. Examples of *Escherichia* bacteria serving as a reference include, as wild-type strains, *Escherichia coli* W3110 (ATCC 27325), *Escherichia coli* MG1655 (ATCC 47076), and so forth, which are derived from a prototype wild-type strain K12.

Furthermore, in the bacterium of the present invention, the activity of an enzyme that catalyzes a reaction branching off of the biosynthetic pathway of L-threonine and producing a compound other than L-threonine may be decreased or deficient. Examples of such an enzyme include threonine dehydrogenase, threonine deaminase, and threonine dehydratase. Strains having these enzymes with decreased or deficient activity are described in International Patent Publications WO95/23864, WO96/17930, WO03/080843, WO04/087895, and so forth.

Furthermore, in the bacterium of the present invention, the activity of an enzyme involved in the glycolytic pathway, TCA cycle, or respiration chain that adversely affects the L-threonine production, an enzyme which regulates gene expression, or an enzyme of a byproduct biosynthesis system may be decreased or made deficient, in addition to an L-threonine degradation system enzyme. Examples of a gene coding for such an enzyme include the gene coding for σ (sigma) factor of RNA polymerase (rpoS, International Patent Publication WO01/05939 and WO03/074719), phosphoenol pyruvate carboxylase gene (pckA, International Patent Publication WO02/29080), phosphoglucose isomerase gene (pgi, Molecular and General Genetics, 217(1): 126-31 (1989)), pyruvate oxidase gene (poxB, International Patent Publication WO02/29080), and so forth.

In order to decrease or eliminate the activity of an enzyme as described above, a mutation that decreases or eliminates intracellular activity of the enzyme can be introduced into the gene of the aforementioned enzyme on a chromosome by a typical mutation treatment, It is achieved by, for example, deleting a gene coding for an enzyme on a chromosome by gene recombination, modifying an expression control sequence such as a promoter and the Shine-Dalgarno (SD) sequence, and so forth. Furthermore, it can also be achieved by introducing one or more amino acid substitutions (missense mutation), introducing a stop codon (nonsense mutation), introducing a frameshift mutation, which adds or deletes one or two nucleotides, or deleting a part or whole region of a gene (Journal of Biological Chemistry, 272: 8611-8617 (1997)) in a region coding for an enzyme on a chromosome. Furthermore, enzymatic activity can also be decreased or eliminated by constructing a gene coding for a mutant enzyme in which a coding region is deleted, and substituting this gene for a normal gene on a chromosome by homologous recombination or the like, or introducing a transposon or IS factor into the gene. Furthermore, it can also be achieved by inhibiting the expression with an antisense-RNA (Proceedings of the National Academy of Sciences, USA, 95 5511-5515 (1998); Journal of Biological Chemistry, 266, 20833-20839 (1991)).

In order to introduce a mutation that decreases or eliminates the activity of an enzyme as mentioned above by gene recombination, for example, the following method is used. A mutant-type gene can be substituted for a target gene on a chromosome by modifying a partial sequence of the target gene to prepare a mutant gene mutated so that a normal functioning enzyme is not produced, and transforming an *Escherichia* bacterium with a DNA containing this gene to cause recombination of the mutant gene and the gene on chromosome. Such site-specific mutation utilizing gene substitution by homologous recombination is known, and examples thereof include a method of using a linear DNA, a method of using a plasmid containing a temperature-sensitive replication origin (U.S. Patent No. 6,303,383; Japanese Patent Laid-open No. 05-007491), and so forth. Furthermore, the aforementioned site-specific mutation by gene substitution utilizing homologous recombination can also be performed by using a plasmid that does not have replication ability in a host.

As a result of decrease in activity or expression by such methods, the enzymatic activity decreases by 75%, 50%, 25%, or 10% as compared with a wild-type strain or unmodified strain, or the activity is completely eliminated. Furthermore, two or more of these methods for decreasing enzyme activity may be implemented in combination, or they may be used in combination with the increase of enzyme activity described above. Examples of an *Escherichia* bacterium serving as a reference include *Escherichia coli* W3110 (ATCC 27325), *Escherichia coli* MG1655 (ATCC 47076), and so forth, which are derived from a prototype wild-type strain K12.

The L-threonine producing bacterium used for the present invention may be a strain which is modified so that it can assimilate sucrose as a carbon source. For example, a strain that assimilates sucrose can be obtained from the *E. coli* H155 strain by P1 transduction, using its ability to grow with sucrose as the sole carbon source as an index. An L-threonine producing bacterium that can assimilate sucrose as a carbon source can be constructed by referring to International Patent Publication WO90/04636.

Furthermore, in addition to the aforementioned gene manipulation, examples of the method for constructing an L-threonine producing bacterium include a method of treating an *Escherichia* bacterium by UV irradiation or with a typical mutabenesis agent such as N-methyl-N'-nitro-N-nitrosoguanidine (NTG) and nitrous acid to obtain strains resistant to an L-amino acid or L-amino acid analogue or L-amino acid auxotrophic strains, and selecting a strain which has improved L-threonine producing ability. Examples thereof include the borrelidin resistant mutant strain (U.S. Patent No. 5,939,307), diaminosuccinic acid resistant mutant strain (International Patent Publication WO00/09661), α-methylserine resistant mutant strain (International Patent Publication WO00/09661 fluoropyruvate resistant mutant strain (International Patent Publication WO00/09661), acetic acid resistant mutant strain (U.S. Patent No. 5,919,670), threonine resistant mutant strain (International Patent Publication WO90/04636), and AHV resistant mutant strain (Genetika, 16: 206 (1978)).

### <3-2> Examples of L-threonine producing bacteria that can be used for the present invention

Examples of *Escherichia* bacteria imparted with an L-threonine producing ability that can be used for the present invention will be described below. However, bacteria that can be used for the present invention are not limited to these examples, so long as the bacterium has an ability to produce L-threonine.

As an example of L-threonine producing bacterium that can be used for the present invention, the *Escherichia coli* VKPM B-3996 strain (U.S. Patent No. 5,175,107) is encompassed. This VKPM B-3996 strain was deposited at Russian National Collection of Industrial Microorganisms (VKPM)-(Russia, 117545 Moscow, 1 Dorozhny proezd. 1) on November 19, 1987 with a registration number of VKPM B-3996. This VKPM B-3996 strain has the plasmid pVIC40 (International Patent Publication WO90/04636) obtained by inserting the threonine biosynthetic genes (threonine operon *thrAB*) into pAYC32, which is a broad host vector plasmid having a streptomycin resistance marker (refer to Chistorerdov, A.Y, Tsygankov, Y.D., Plasmid, 1986, 16, 161-167). In pVIC40, feedback inhibition of aspartokinase I-homoserine dehydrogenase I encoded by *thrA* in the threonine operon by L-threonine is eliminated.

As an example of L-threonine producing bacterium that can be used for the present invention, the *Escherichia coli* VKPM B-5318 strain (European Patent No.0593792) is also encompassed. The VKPM B-5318 strain was deposited at Russian National Collection of Industrial Microorganisms (VKPM) (Russia, 117545 Moscow, 1 Dorozhny proezd. 1) on May 3, 1990 with a registration number of VKPM B-5318. This VKPM B-5318 strain does not require isoleucine, has the temperature-sensitive C1 repressor, PR promoter, and the threonine operon. That is, the threonine biosynthesis-related genes, in which the attenuator region and the native transcription regulating region are deleted, and are located downstream from the gene for the N-terminus region of the Cro protein of lambda phage. Furthermore, this strain contains a recombinant plasmid DNA which has been constructed so that expression of the threonine biosynthesis-involved genes are regulated by the repressor and promoter of lambda phage.

The *Escherichia coli* 427T23 (U.S. Patent No. 5,631,157) can also be exemplified as a preferred example of the L-threonine producing bacterium. The 427T23 strain has homoserine dehydrogenase which is not subject to feedback inhibition by L-threonine. Also, this strain has attenuated threonine deaminase activity and can use sucrose as a carbon source. The 427T23 strain has been deposited at American Type Culture Collection with an accession number of ATCC 98082.

The *Escherichia coli* kat-13 (U.S. Patent No. 5,175,107) can also be preferably used as the L-threonine producing bacterium. The kat-13 strain is resistant to borrelidin, has homoserine dehydrogenase which is not subject to feedback inhibition by L-threonine, attenuated threonine deaminase activity, and can use sucrose as a carbon source.

The *Escherichia coli* TDH6 strain (Japanese Patent Laid-open No. 2001-346578) transformed with genes coding for threonine biosynthesis enzymes can also be preferably used as the L-threonine producing bacterium. The TDH6 strain is deficient in threonine dehydrogenase activity, and was obtained by removing pVIC40 that carries the genes coding for the threonine biosynthesis enzymes from the B-3996 strain (US5,631,157), and has been deposited at Russian National Collection of Industrial Microorganisms (VKPM), (Russia, 117545 Moscow, 1 Dorozhny proezd. 1) with an accession number of VKPM B-3420.

Furthermore, other examples of the L-threonine producing bacterium that can be used for the present invention include the following bacterial strains:
*Escherichia coli* MG-442 (CMIMB-1628, U.S. Patent No. 4,278,765)
*Escherichia coli* VL334/pYN7 (U.S. Patent No. 4,278,765)
*Escherichia coli* H-4225 (FERM BP-1236, U.S. Patent No. 5,017,483)
*Escherichia coli* H-7256 (FERM BP-2137)
*Escherichia coli* DSM9807 (KCCM-10168)

### Examples

The present invention will be explained more specifically with reference to the following examples. However, the present invention is not limited to these examples.

### Example 1: Effect of regulation of initially added sulfur in fed-batch culture

First, the effect of limiting the sulfur in the initial medium of the main culture to a predetermined concentration or lower during L-threonine production was examined in a fed-batch culture.

The VKPM B-5318 strain was cultured on an LB agar medium plate (10 g/L of trypton, 5 g/L of yeast extract, 5 g/L of NaCl, 15 g/L of agar) containing 20 mg/L of streptomycin sulfate at 37°C for 24 hours, and 1/10 of the cells on the plate were scraped off from one plate and inoculated into 50 mL of LB medium (10 g/L of trypton, 5 g/L of yeast extract, 5 g/L of NaCl) containing 20 mg/L of streptomycin sulfate in a baffle flask to perform a seed culture at 40°C and 144 rpm for 6 hours.

After completion of the seed culture, the seed culture medium in a volume equivalent to 16% of the main culture medium volume was inoculated into a 1-L jar fermenter charged with 300 mL of the main culture medium, and culture was performed at 40°C and pH 7.0. The main culture medium composition is shown below.
Main culture medium composition:

| | |
|---|---|
| Sucrose | 27 g/L |
| Yeast extract | 1.8 g/L |
| KH₂PO₄ | 1.5 g/L |
| NaCl | 0.6 g/L |
| MgSO₄·7H₂O | 0.36 g/L |
| FeSO₄·7H₂O | 18 mg/L |
| MnSO₄·4H₂O | 18 mg/L |
| Streptomycin sulfate | 20 mg/L |

Ammonium sulfate was appropriately added so that the initially added sulfur content is 0.78 to 0.10 g/L in terms of sulfur content.

The culture was adjusted to pH 7.0 during the culture by adding ammonia gas. After the saccharide in the medium was consumed, 600 g/L of a sucrose aqueous solution was added to perform a fed-batch culture.

After 42 hours of the culture, the L-threonine concentration was determined by HPLC.

As a result, the L-threonine fermentation yield increased with a decrease in sulfur, and it markedly increased, in particular, at a sulfur concentration of 0.29 g/L or lower as shown in Table 1.

**Table 1**

| Initially added (NH₄)₂SO₄ (g/L) | Initially added S (g/L) | Yield (%) |
|---|---|---|
| 3.0 | 0.78 | 35.7 |
| 2.0 | 0.54 | 36.6 |
| 1.0 | 0.29 | 38.6 |
| 0.8 | 0.25 | 39.9 |
| 0.4 | 0.15 | 40.8 |
| 0.2 | 0.10 | 43.6 |

### Example 2: Effect of regulation of sulfur in the feed medium in a fed-batch culture

First, in the same manner as in Example 1, the VKPM B-5318 strain was cultured on a LB agar medium plate (10 g/L of trypton, 5 g/L of yeast extract, 5 g/L of NaCl, 15 g/L of agar) containing 20 mg/L of streptomycin sulfate at 37°C for 24 hours, and 1/10 of the bacterial-cells on the plate were scraped off from one plate, and inoculated into 50 mL of LB medium (10 g/L of trypton, 5 g/L of yeast extract, 5 g/L of NaCl) containing 20 mg/L of streptomycin sulfate in a baffle flask to perform seed culture at 40°C and 144 rpm for 6 hours.

After completion of the seed culture, the seed culture medium in a volume equivalent to 16% of the main culture medium volume was inoculated into a 1-L jar fermenter charged with 300 mL of the main culture medium, and culture was performed at 40°C and pH 7.0. The main culture medium had the same composition as that used in Example 1.

The culture was adjusted to pH 7.0 during the culture by adding ammonia gas. After the saccharide in the medium was consumed, fermentation was performed with addition of a feed medium containing sucrose, and adjusted so that the sulfur concentration in the fermentation medium is at a predetermined level or lower. The results are shown in Table 2. The sulfur concentration in the feed medium was controlled to be 0 to 1.22 g/L, and the culture was controlled so that the fermentation medium contains 0.144 to 0.548 g/L of sulfur throughout the entire main culture period.

As a result, it was confirmed that the yield of L-threonine was improved also in fed-batch culture by regulating the sulfur concentration in the fermentation medium to 0.35g/L or lower.

**Table 2**

| (NH₄)₂SO₄ in feed solution (g/L) | S in feed solution (g/L) | Total S (g/L) | Yield (%) |
|---|---|---|---|
| 0.0 | 0.00 | 0.144 | 41.8 |
| 1.0 | 0.24 | 0.225 | 39.1 |
| 2.0 | 0.49 | 0.305 | 38.8 |
| 5.0 | 1.22 | 0.548 | 37.5 |

### Industrial Applicability

According to the present invention, fermentation yield and production of L-threonine can be improved in a method for producing L-threonine by fermentation using a microorganism belonging to the genus *Escherichia* that has an L-threonine producing ability.

## Claims

1. A method for producing L-threonine comprising:
A) culturing a microorganism belonging to the genus Escherichia that has an ability to produce L-threonine, in a fermentation medium containing a carbon source, a nitrogen source, and a sulfur source,
B) collecting L-threonine from the culture, wherein the sulfur concentration in the medium is regulated so that it is 0.35 g/L or lower.

2. The method according to claim 1, wherein the microorganism is Escherichia coli.

3. The method according to any one of claim 1 or 2, wherein an L- threonine biosynthesis enzyme in the microorganism is modified so that the enzyme is not subject to feedback inhibition by L-threonine.

4. The method according to claim 3, wherein the L-threonine biosynthesis enzyme is selected from the group consisting of aspartokinase, homoserine kinase, threonine synthase, and combinations thereof.

5. The method according to any one of claims 1 to 4, wherein the sulfur source is selected from the group consisting of sulfates, thiosulfates, sulfites, cysteine, cystine, glutathione, and combinations thereof.

6. The method according to any one of claims 1 to 5, wherein the culture method is selected from the group consisting of a batch culture method, a fed- batch culture method, and a continuous culture method.

7. The method according to claim 6, wherein the culture method is a fed- batch culture method or a continuous culture method, and wherein a feed medium containing a sulfur source is added to the culture in a fermenter.

8. The method according to claim 7, wherein said feed medium further contains a carbon source and a nutrient having a growth promoting effect, and wherein said feed medium is added to the culture in the fermenter continuously or intermittently so that the concentration of the carbon source in the culture is maintained at 30 g/L or lower after the end of the logarithmic growth of the microorganism.

9. A method for producing animal feed additive based on fermentation broth comprising:
A) culturing a microorganism belonging to the genus Escherichia that has an ability to produce L-threonine, in a fermentation medium containing a carbon source, a nitrogen source, and a sulfur source,
B) conducting the fermentation wherein the sulfur concentration in the medium is regulated so that it is 0.35 g/L or lower,
C) drying the crude fermentation broth to a water content of 10% or less by weight.

10. The method according to claim 9, wherein the microorganism is Escherichia coli.

11. The method according to any one of claim 9 or 10, wherein an L- threonine biosynthesis enzyme in the microorganism is modified so that the enzyme is not subject to feedback inhibition by L-threonine.

12. The method according to claim 11, wherein the L-threonine biosynthesis enzyme is selected from the group consisting of aspartokinase, homoserine kinase, threonine synthase, and combinations thereof.

13. The method according to any one of claims 9 to 12, wherein the sulfur source is selected from the group consisting of sulfates, thiosulfates, sulfites, cysteine, cystine, glutathione, and combinations thereof.

14. The method according to any one of claims 9 to 13, wherein the culture method is selected from the group consisting of a batch culture method, a fed-batch culture method, and a continuous culture method.

15. The method according to claim 14, wherein the culture method is a fed-batch culture method or a continuous culture method, and wherein a feed medium containing a sulfur source is added to the culture in a fermenter.

16. The method according to claim 14, wherein said feed medium further contains a carbon source and a nutrient having a growth promoting effect, and wherein said feed medium is added to the culture in the fermenter continuously or intermittently so that the concentration of the carbon source in the culture is maintained at 30 g/L or lower after the end of the logarithmic growth of the microorganism.

17. The method according to any one of claims 9 to 16, wherein more than 95% L-threonine is included in said feed additive.

## Patentansprüche

1. Verfahren zum Herstellen von L-Threonin, umfassend:
(A) Kultivieren eines zu der Gattung Escherichia gehörenden Mikroorganismuses, der eine Fähigkeit aufweist, L-Threonin herzustellen, in einem Fermentationsmedium, das eine Kohlenstoffquelle, eine Stickstoffquelle und eine Schwefelquelle enthält,
(B) Sammeln von L-Threonin aus der Kultur,
wobei die Schwefelkonzentration in dem Medium reguliert ist, so dass sie 0,35 g/l oder niedriger ist.

2. Verfahren gemäß Anspruch 1, worin der Mikroorganismus Escherichia coli ist.

3. Verfahren gemäß Anspruch 1 oder 2, worin ein Enzym der Biosynthese von L-Threonin im Mikroorganismus modifiziert ist, so dass das Enzym keiner Rückkopplungshemmung durch L-Threonin unterworfen ist.

4. Verfahren gemäß Anspruch 3, worin das Enzym der Biosynthese von L-Threonin ausgewählt ist aus der Gruppe, bestehend aus Aspartokinase, Homoserinkinase, Threoninsynthase und Kombinationen davon.

5. Verfahren gemäß irgendeinem der Ansprüche 1 bis 4, worin die Schwefelquelle ausgewählt ist aus der Gruppe, bestehend aus Sulfaten, Thiosulfaten, Sulfiten, Cystein, Cystin, Glutathion und Kombinationen davon.

6. Verfahren gemäß irgendeinem der Ansprüche 1 bis 5, worin das Kultivierungsverfahren ausgewählt ist aus der Gruppe, bestehend aus einem Batch-Kultivierungsverfahren, einem Fed-batch-Kultivierungsverfahren und einem Verfahren mit kontinuierlicher Kultivierung.

7. Verfahren gemäß Anspruch 6, worin das Kultivierungsverfahren ein Fed-batch-Kultivierungsverfahren oder ein Verfahren mit kontinuierlicher Kultivierung ist, und worin ein Nährmedium, das eine Schwefelquelle enthält, zu der Kultur in einem Fermenter hinzugegeben wird.

8. Verfahren gemäß Anspruch 7, worin das Nährmedium ferner eine Kohlenstoffquelle und eine Nährsubstanz enthält, die eine wachstumsfördernde Wirkung aufweist, und worin das Nährmedium kontinuierlich oder in Abständen zu der Kultur im Fermenter hinzugegeben wird, so dass die Konzentration der Kohlenstoffquelle in der Kultur bei 30 g/l oder niedriger nach dem Ende des logarithmischen Wachstums des Mikroorganismuses gehalten wird.

9. Verfahren zum Herstellen eines auf einer Fermentationsbrühe basierenden Tierfutterzusatzes, umfassend:
(A) Kultivieren eines zu der Gattung Escherichia gehörenden Mikroorganismuses, der eine Fähigkeit aufweist, L-Threonin herzustellen, in einem Fermentationsmedium, das eine Kohlenstoffquelle, eine Stickstoffquelle und eine Schwefelquelle enthält,
(B) Durchführen der Fermentation, wobei die Schwefelkonzentration in dem Medium reguliert ist, so dass sie 0,35 g/l oder niedriger ist,
(C) Trocknen der rohen Fermentationsbrühe auf einen Wassergehalt von 10 Gew.% oder weniger.

10. Verfahren gemäß Anspruch 9, worin der Mikroorganismus Escherichia coli ist.

11. Verfahren gemäß Anspruch 9 oder 10, worin ein Enzym der Biosynthese von L-Threonin im Mikroorganismus modifiziert ist, so dass das Enzym keiner Rückkopplungshemmung durch L-Threonin unterworfen ist.

12. Verfahren gemäß Anspruch 11, worin das Enzym der Biosynthese von L-Threonin ausgewählt ist aus der Gruppe, bestehend aus Aspartokinase, Homoserinkinase, Threoninsynthase und Kombinationen davon.

13. Verfahren gemäß irgendeinem der Ansprüche 9 bis 12, worin die Schwefelquelle ausgewählt ist aus der Gruppe, bestehend aus Sulfaten, Thiosulfaten, Sulfiten, Cystein, Cystin, Glutathion und Kombinationen davon.

14. Verfahren gemäß irgendeinem der Ansprüche 9 bis 13, worin das Kultivierungsverfahren ausgewählt ist aus der Gruppe, bestehend aus einem Batch-Kultivierungsverfahren, einem Fed-batch-Kultivierungsverfahren und einem Verfahren mit kontinuierlicher Kultivierung.

15. Verfahren gemäß Anspruch 14, worin das Kultivierungsverfahren ein Fed-batch-Kultivierungsverfahren oder ein Verfahren mit kontinuierlicher Kultivierung ist, und worin ein Nährmedium, das eine Schwefelquelle enthält, zu der Kultur in einem Fermenter hinzugegeben wird.

16. Verfahren gemäß Anspruch 14, worin das Nährmedium ferner eine Kohlenstoffquelle und eine Nährsubstanz enthält, die eine wachstumsfördernde Wirkung aufweist, und worin das Nährmedium kontinuierlich oder in Abständen zu der Kultur im Fermenter hinzugegeben wird, so dass die Konzentration der Kohlenstoffquelle in der Kultur bei 30 g/l oder niedriger nach dem Ende des logarithmischen Wachstums des Mikroorganismuses gehalten wird.

17. Verfahren gemäß irgendeinem der Ansprüche 9 bis 16, worin mehr als 95 % an L-Threonin in dem Futterzusatz eingeschlossen sind.

## Revendications

1. Procédé pour produire de la L-thréonine comprenant :
A) la culture d'un micro-organisme appartenant au genre Escherichia qui a une aptitude à produire de la L-thréonine, dans un milieu de fermentation contenant une source de carbone, une source d'azote et une source de soufre,
B) la collecte de la L-thréonine à partir de la culture, où la concentration de soufre dans le milieu est régulée de sorte qu'elle est 0,35 g/L ou moins.

2. Procédé selon la revendication 1 où le micro-organisme est Escherichia coli.

3. Procédé selon l'une quelconque des revendications 1 et 2 où une enzyme de biosynthèse de L-thréonine dans le micro-organisme est modifiée de sorte que l'enzyme n'est pas soumise à une rétro-inhibition par la L-thréonine.

4. Procédé selon la revendication 3 où l'enzyme de biosynthèse de L-thréonine est choisie dans le groupe consistant en aspartokinase, homosérine kinase, thréonine synthase et leurs combinaisons.

5. Procédé selon l'une quelconque des revendications 1 à 4 où la source de soufre est choisie dans le groupe consistant en les sulfates, les thiosulfates, les sulfites, la cystéine, la cystine, le glutathion et leurs combinaisons.

6. Procédé selon l'une quelconque des revendications 1 à 5 où le procédé de culture est choisi dans le groupe consistant en un procédé de culture discontinu, un procédé de culture discontinu alimenté et un procédé de culture continu.

7. Procédé selon la revendication 6 où le procédé de culture est un procédé de culture discontinu alimenté ou un procédé de culture continu, et où un milieu d'alimentation contenant une source de soufre est ajouté à la culture dans un fermenteur.

8. Procédé selon la revendication 7 où ledit milieu d'alimentation contient en outre une source de carbone et un nutriment ayant un effet favorisant la croissance, et où ledit milieu d'alimentation est ajouté à la culture dans le fermenteur en continu ou par intermittence de sorte que la concentration de la source de carbone dans la culture est maintenue à 30 g/L ou moins après la fin de la croissance logarithmique du micro-organisme.

9. Procédé pour produire un additif alimentaire pour animaux basé sur un bouillon de fermentation comprenant :
A) la culture d'un micro-organisme appartenant au genre Escherichia qui a une aptitude à produire de la L-thréonine, dans un milieu de fermentation contenant une source de carbone, une source d'azote et une source de soufre,
B) la conduite de la fermentation où la concentration de soufre dans le milieu est régulée de telle sorte qu'elle est 0,35 g/L ou moins,
C) le séchage du bouillon de fermentation brut jusqu'à une teneur en eau de 10 % ou moins en masse.

10. Procédé selon la revendication 9 où le micro-organisme est Escherichia coli.

11. Procédé selon l'une quelconque des revendications 9 et 10 où une enzyme de biosynthèse de L-thréonine dans le micro-organisme est modifiée de sorte que l'enzyme n'est pas soumise à une rétro-inhibition par la L-thréonine.

12. Procédé selon la revendication 11 où l'enzyme de biosynthèse de L-thréonine est choisie dans le groupe consistant en aspartokinase, homosérine kinase, thréonine synthase et leurs combinaisons.

13. Procédé selon l'une quelconque des revendications 9 à 12 où la source de soufre est choisie dans le groupe consistant en les sulfates, les thiosulfates, les sulfites, la cystéine, la cystine, le glutathion et leurs combinaisons.

14. Procédé selon l'une quelconque des revendications 9 à 13 où le procédé de culture est choisi dans le groupe consistant en un procédé de culture discontinu, un procédé de culture discontinu alimenté et un procédé de culture continu.

15. Procédé selon la revendication 14 où le procédé de culture est un procédé de culture discontinu alimenté ou un procédé de culture continu, et où un milieu d'alimentation contenant une source de soufre est ajouté à la culture dans un fermenteur.

16. Procédé selon la revendication 14 où ledit milieu d'alimentation contient en outre une source de carbone et un nutriment ayant un effet favorisant la croissance, et où ledit milieu d'alimentation est ajouté à la culture dans le fermenteur en continu ou par intermittence de sorte que la concentration de la source de carbone dans la culture est maintenue à 30 g/L ou moins après la fin de la croissance logarithmique du micro-organisme.

17. Procédé selon l'une quelconque des revendications 9 à 16 où plus de 95 % de L-thréonine sont inclus dans ledit additif alimentaire.
